# EUROPÄISCHE PATENTSCHRIFT

(11) **EP 2 245 026 B1**
(45) Veröffentlichungstag und Bekanntmachung des Hinweises auf die Patenterteilung: **01.08.2012**
(21) Anmeldenummer: 09707435.5
(22) Anmeldetag: 05.02.2009
(51) Int. Cl.: C07D 403/12, A61K 31/517, A61P 11/00

(54) **SPIROCYCLISCHE HETEROCYCLEN, DIESE VERBINDUNGEN ENTHALTENDE ARZNEIMITTEL, DEREN VERWENDUNG UND VERFAHREN ZU IHRER HERSTELLUNG**
SPIROCYCLIC HETEROCYCLES, MEDICAMENTS CONTAINING SAID COMPOUNDS, USE THEREOF AND METHOD FOR THEIR PRODUCTION
HÉTÉROCYCLES SPIROCYCLIQUES, MÉDICAMENTS CONTENANT CES COMPOSÉS, LEUR UTILISATION ET PROCÉDÉ POUR LES PRODUIRE

(30) Priorität: 07.02.2008 EP 08101353
(43) Veröffentlichungstag der Anmeldung: 03.11.2010
(73) Patentinhaber: Boehringer Ingelheim International GmbH, 55216 Ingelheim am Rhein (DE)
(72) Erfinder: HIMMELSBACH, Frank, 55216 Ingelheim Am Rhein (DE); JUNG, Birgit, 55216 Ingelheim Am Rhein (DE); LOTZ, Ralf, 55216 Ingelheim Am Rhein (DE)
(74) Vertreter: Hammann, Heinz
(86) Internationale Anmeldenummer: PCT/EP2009/000805
(87) Internationale Veröffentlichungsnummer: WO 2009/098061

(56) Entgegenhaltungen:
- WO-A-03/082290
- WO-A-2006/034015

## Beschreibung

Gegenstand der vorliegenden Erfindung sind spirocyclische Heterocyclen der allgemeinen Formel 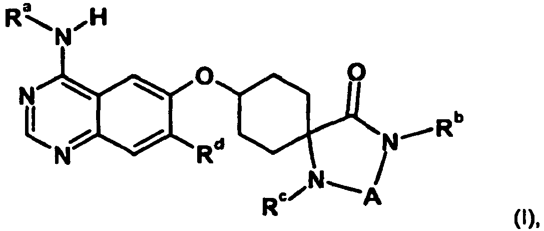 deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

Aufgabe der vorliegenden Erfindung ist es, neue Verbindungen bereitzustellen, die aufgrund ihrer pharmazeutischen Wirksamkeit als Tyrosin-Kinase Hemmer zur Anwendung auf therapeutischem Gebiet, d.h. zur Behandlung von pathophysiologischen Prozessen, die durch Überfunktion von Tyrosinkinasen hervorgerufen werden, gelangen können.
Tyrosinkinaseinhibitoren mit ähnlicher Struktur sind beispielsweise aus der WO03/082290 bekannt.

### Detaillierte Beschreibung der Erfindung

Überraschenderweise wurde gefunden, dass die vorstehend genannte Aufgabe durch Verbindungen der Formel (I), worin die Reste **R^{a}** bis **R^{d}** und **A** die nachstehend genannten Bedeutungen haben, gelöst wird.
Die vorliegende Erfindung betrifft daher Verbindungen der allgemeinen Formel (I), 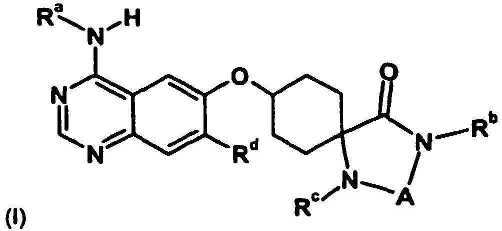 worin
- **R^{a}**: eine Phenyl- oder 1-Phenylethyl-Gruppe, in denen der Phenylkern jeweils durch die Reste **R¹** bis **R³** substituiert ist, wobei
**R¹ und R²** gleich oder verschieden, Wasserstoff oder einen Rest ausgewählt aus der Gruppe bestehend aus F, Cl, Br, I, OCH₂F, OCHF₂, OCF₃, CH₂F, CHF₂, CF₃, CN, NO₂, NH₂ und OH,
oder
einen Rest ausgewählt aus der Gruppe bestehend aus C₁₋₄-Alkyl, C₁₋₄-Alkyl-O-, C₂₋₃-Alkenyl, C₂₋₃-Alkinyl, Phenyl, Phenyl-O-, Phenyl-C₁₋₃-alkyl- und Phenyl-C₁₋₃-alkyl-O-, Heteroaryl, Heteroaryl-O-, Heteroaryl-C₁₋₃-alkyl- und Heteroaryl-C₁₋₃-alkyl-O, wobei die vorstehend genannten Phenylgruppen durch Reste **R⁵**, mono- oder di- substituiert sind,
**und**
**R³** Wasserstoff, oder einen Rest ausgewählt aus der Gruppe bestehend aus F, Cl, Br und CH₃.
**R^{b}** Wasserstoff, oder einen Rest, gegebenenfalls substituiert, ausgewählt aus der Gruppe bestehend aus C₁₋₆-Alkyl, C₃₋₆-Cycloalkyl- und C₃₋₆-Cycloalkyl-C₁₋₃-alkyl-,
- **R^{c}**: Wasserstoff, oder einen Rest, gegebenenfalls substituiert, ausgewählt aus der Gruppe bestehend aus C₁₋₆-Alkyl, C₃₋₆-Cycloalkyl-, G₃₋₆-Cycloalkyl-C₁₋₃-alkyl, C₁₋₆-Alkyl-CO-, C₃₋₆-Cycloalkyl-CO-, C₃₋₆-Cycloalkyl-C₁₋₃-alkyl-CO-. C₁₋₆-Alkyl-SO₂-, C₃₋₆-Cycloalkyl-SO₂-, C₃₋₆-Cycloalkyl-C₁₋₃-alkyl-SO₂-, Phenyl-CO- und Phenyl-SO₂-,
- **R^{d}**: Wasserstoff oder einen Rest ausgewählt aus der Gruppe bestehend aus F, Cl, Br, I, OH, C₁₋₄-Alkyl, C₁₋₄-Alkyl-O-, mit 1 bis 3 Fluoratomen substituiertes C₁₋₂-Alkyl-O-, C₃₋₇-Cycloalkyl-O-, C₃₋₇-Cycloalkyl-C₁₋₄-alkyl-O-, Tetrahydrofuran-3-yl-O-, Tetrahydropyran-3-yl-O-, Tetrahydro-pyran-4-yl-O-, Tetrahydrofuranyl-C₁₋₄-alkyl-O- und Tetrahydropyranyl-C₁₋₄-alkyl-O-,
oder
R⁴-C₁₋₄-alkyl-, wobei die Verknüpfung der Reste **R⁴** über jedes C-Atom des Alkylrestes erfolgen kann,
oder
R⁴-C₂₋₄-alkyl-O-, wobei der Rest R⁴ durch mindestens 2 C-Atome von dem Sauerstoffatom getrennt ist,
oder
einen Rest ausgewählt aus der Gruppe bestehend aus Pyrrolidin-2-yl-C₁₋₄-alkyl-O-, Pyrrolidin-3-yl-C₁₋₄-alkyl-O-, Piperidin-2-yl-C₁₋₄-alkyl-O-, Piperidin-3-yl-C₁₋₄-alkyl-O-, Piperidin-4-yl-C₁₋₄-alkyl-O-, Azepan-2-yl-C₁₋₄-alkyl-O-, Azepan-3-yl-C₁₋₄-alkyl-O-, Azepan-4-yl-C₁₋₄-alkyl-O-, Morpholin-2-yl-C₁₋₄-alkyl-O-, Morpholin-3-yl-C₁₋₄-alkyl-O-, 1-(C₁₋₃-Alkyl)-pyrrolidin-2-yl-C₁₋₄-alkyl-O-, 1-(C₁₋₃-Alkyl)-pyrrolidin-3-yl-C₁₋₄-alkyl-O-, 1-(C₁₋₃-Alkyl)-piperidin-2-yl-C₁₋₄-alkyl-O-, 1-(C₁₋₃-Alkyl)-piperidin-3-yl-C₁₋₄-alkyl-O-, 1-(C₁₋₃-Alkyllpiperidin-4-yl-C₁₋₄-alkyl-O-, 1-(C₁₋₃-Alkyl)-azepan-2-yl-C₁₋₄-alkyl-O-, 1-(C₁₋₃-Alkyl)-azepan-3-yl-C₁₋₄-alkyl-O-, 1-(C₁₋₃-Alkyl)-azepan-4-yl-C₁₋₄-alkyl-O-, 4-(C₁₋₃-Alkyl)-morpholin-2-yl-C₁₋₄-alkyl-O- und 4-(C₁₋₃-Alkyl)-morpholin-3-yl-C₁₋₄-alkyl-O-,
wobei
- **R⁴**: einen Rest, gleich oder verschieden, ausgewählt aus der Gruppe bestehend aus OH, C₁₋₃-Alkyl-O-, C₃₋₆-Cycloalkyl-O-, NH₂, C₁₋₃-Alkyl-NH-, (C₁₋₃-Alkyl)₂N-, (2-Methoxyethyl)₂N-, Pyrrolidin-1-yl-, Piperidin-1-yl-, Azepan-1-yl-, Morpholin-4-yl-, 1,4-Oxazepan-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo[3.2.1]oct-3-yl-, Piperazin-1-yl-, 4-(C₁₋₃-Alkyl)-piperazin-1-yl-, 1,4-Diazepan-1-yl-, 4-(C₁₋₃-Alkyl)-1,4-diazepan-1-yl-, HCO-NH-, C₁₋₄-Alkyl-CO-NH-, C₁₋₃-Alkyl-O-C₁₋₃-alkyl-CO-NH-, C₁₋₄-Alkyl-O-CO-NH-, H₂NCONH-, C₁₋₃-Alkyl-NH-CO-NH-, (C₁₋₃-Alkyl)₂-N-CONH-, Pyrrolidin-1-yl-CO-NH-, Piperidin-1-yl-CO-NH -, Piperazin-1-yl-CO-NH-, 4-(C₁₋₃-Alkyl)-piperazin-1-yl-CO-NH -, Morpholin-4-yl-CO-NH- und C₁₋₄-Alkyl-SO₂-NH-,

wobei die bei der Definition des Restes **R^{d}** vorstehend genannten Pyrrolidinyl-, Piperidinyl-, Azepan-1-yl-, Piperazinyl-, 1,4-Diazepan-1-yl-, Morpholinyl- und 1,4-Oxazepan-4-yl-Gruppen jeweils zusätzlich durch eine oder zwei C₁₋₃-Alkylgruppen substituiert sein können,
und
wobei die vorstehend genannten Phenylgruppen durch Reste **R⁵** mono- oder di- substituiert sind, wobei
- **R⁵**: Wasserstoff, oder einen Rest, gleich oder verschieden, ausgewählt aus der Gruppe bestehend aus F, Cl, Br, I, OH, CN, C₁₋₃-Alkyl-, C₁₋₃-Alkyl-O-, CHF₂, CF₃, -O-CHF₂ und -O-CF₃,
und
soweit nichts anderes erwähnt wurde, die vorstehend erwähnten Alkylgruppen geradkettig oder verzweigt sein können,
- **A**: -CO- oder -C₁-C₃-alkylen-, wobei der -C₁-C₃-alkylen- Rest durch einen Rest **R⁶** 1-, 2-, 3- oder 4-fach substituiert sein kann, und
- **R⁶**: gleich oder verschieden, Wasserstoff, oder einen Rest ausgewählt aus der Gruppe bestehend aus OH, C₁-C₄-Alkyl und -O-C₁-C₄-Alkyl
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate und Hydrate,
bedeuten.

Bevorzugt sind Verbindungen der Formel (I), worin
- **R^{a}**: einen Rest ausgewählt aus der Gruppe bestehend aus 3-Chlor-2-fluor-phenyl-, 3-Chlor-4-fluor-phenyl-, 5-Chlor-2-fluor-phenyl-, 2-Fluor-3-methyl-phenyl-, 2-Fluor-5-methylphenyl-, 4-Fluor-3-methyl-phenyl- und 3-Chlor-2-methyl-phenyl-,
- **R^{b} und R^{c}**: gleich oder verschieden, Wasserstoff oder C₁₋₃-Alkyl,
- **R^{d}**: C₁₋₃-Alkyl-O-,
soweit nichts anderes erwähnt wurde, die vorstehend erwähnten Alkylgruppen geradkettig oder verzweigt sein können,
- **A**: -CH₂CH₂-, wobei der -CH₂CH₂- Rest durch eine 1 oder 2 Methylgruppen substituiert sein kann,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate und Hydrate, bedeuten.

Ein weiterer Gegenstand der Erfindung sind Verbindungen der Formel (I) zur Verwendung als Arzneimittel.

Bevorzugt ist die Verwendung der Verbindungen der Formel (I), wobei es sich um entzündliche oder allergische Erkrankungen der Atemwege handelt.

Besonders bevorzugt ist die Verwendung der Verbindungen der Formel (I), wobei es sich um eine Erkrankung handelt, die ausgewählt ist aus der Gruppe bestehend aus chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen, Alveolitis, hyperreaktiver Atemwege, Nasenpolypen, Lungenödemen, Pneumonitis aufgrund unterschiedlicher Genese wie Strahlen-induziert oder durch Aspiration oder infektiöse, Kollagenosen wie Lupus eryth, systemische Sklerodermie, Sarkoidose und M. Boeck, sowie zur Behandlungen von Exazerbationen bei Asthma und COPD, die viral, bakteriell oder durch andere Ursachen ausgelöst wurden, zur Behandlung von viralen oder bakteriellen Infektionen der Atemwege oder Lunge.

Weiterhin bevorzugt ist die Verwendung der Verbindungen der Formel (I), wobei es sich um entzündliche oder allergische Krankheitszustände handelt, bei denen Autoimmun-Reaktionen beteiligt sind.

Weiterhin bevorzugt ist die Verwendung der Verbindungen der Formel (I), wobei es sich um eine Erkrankung in Form von benignen oder malignen Tumoren handelt.

Ein weiterer Gegenstand der Erfindung ist eine pharmazeutische Formulierung enthaltend eine Verbindung der Formel (I).

Bevorzugt ist eine oral applizierbare pharmazeutische Formulierung enthaltend eine Verbindung der Formel (I).

Ein weiterer Gegenstand der Erfindung ist eine Arzneimittelkombinationen, die neben einer oder mehrerer Verbindungen der Formel (I) als weiteren Wirkstoff einen oder mehrere Verbindungen enthalten, die ausgewählt sind aus den Klassen der Betamimetika, Anticholinergika, Corticosteroiden, weitere PDE4-Inhibitoren, LTD4-Antagonisten, EGFR-Hemmem, Dopamin-Agonisten, H1-Antihistaminika, PAF-Antagonisten und PI3-Kinase Inhibitoren oder zwei- oder dreifach Kombinationen davon.

### Verwendete Begriffe und Definitionen

Unter dem Begriff "gegebenenfalls substituiert" wird im Rahmen der Erfindung die genannte Gruppe verstanden, die gegebenenfalls mit einem niedermolekularen Rest substituiert ist. Als niedermolekulare Reste werden als chemisch sinnvoll anzusehende Gruppen verstanden, bestehend aus 1-25 Atomen. Bevorzugt haben solche Gruppen keinen negativen Effekt auf die pharmakologische Wirksamkeit der Verbindungen.

Beispielsweise können die Gruppen umfassen:
_{•} Gerade oder verzweigte Kohlenstoffketten, gegebenenfalls unterbrochen durch Heteroatome, gegebenenfalls substituiert mit Ringen, Heteroatomen oder anderen gängigen funktionellen Gruppen.
• Aromatische oder nicht-aromatische Ringsysteme bestehend aus Kohlenstoffatomen und gegebenenfalls Heteroatomen, die wiederum substituiert sein können mit funktionellen Gruppen.
• Mehrere aromatische oder nicht-aromatische Ringsysteme bestehend aus Kohlenstoffatomen und gegebenenfalls Heteroatomen, die durch eine oder mehrere Kohlenstoffketten, gegebenenfalls unterbrochen durch Heteroatome, gegebenenfalls substituiert mit Heteroatomen oder anderen gängigen funktionellen Gruppen verknüpft sein können.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Sofern in der Strukturformel eines Substituenten ein einseitig offener Bindestrich "-" verwendet wird, ist dieser Bindestrich als Verknüpfungspunkt zum Rest des Moleküls zu verstehen. Der Substituent tritt an die Stelle der entsprechenden Reste **R^{a}**, **R^{b}**, etc.. Sofern in der Bezeichnung oder Strukturformel eines Substituenten kein einseitig offener Bindestrich verwendet wird, ergibt sich der Verknüpfungspunkt zum Rest des Moleküls eindeutig aus der Bezeichnung oder Strukturformel selbst.

Verbindungen der allgemeinen Formel (I) können Säuregruppen besitzen, hauptsächlich Carboxylgruppen, und/oder basische Gruppen wie z.B. Aminofunktionen. Verbindungen der allgemeinen Formel (I) können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen. Zur Darstellung der Alkali- und Erdalkalimetallsalze der Verbindung der Formel (I), kommen vorzugsweise die Alkali- und Erdalkalihydroxide und - hydride in Betracht, wobei die Hydroxide und Hydride der Alkalimetalle, besonders des Natriums und Kaliums bevorzugt, Natrium- und Kaliumhydroxid besonders bevorzugt sind. (siehe auch Pharmaceutical salts, Birge, S.M. et al., J. Pharm. Sci., (1977), 66, 1-19)

Wie vorstehend genannt, können die Verbindungen der allgemeinen Formel (I) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen Diastereomeren, Mischungen der einzelnen Diastereomeren und/oder der einzelnen Enantiomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organischen Säuren - wie beispielsweise Weinsäure, Fumarsäure, Zitronensäure oder Methansulfonsäure.

"Schutzgruppen" im Sinne der vorliegenden Erfindung sind als Sammelbezeichnung für solche organische Reste zu verstehen, mit denen bestimmte funktionelle Gruppen eines mehrere aktive Zentren enthaltenden Moleküls vorübergehend gegen den Angriff von Reagenzien geschützt werden können, so dass Reaktionen nur an den gewünschten (ungeschützten) Stellen stattfinden. Die Schutzgruppen sollen unter milden Bedingungen selektiv einzuführen sein. Sie müssen für die Dauer des Schutzes unter allen Bedingungen der durchzuführenden Reaktionen und Reinigungsoperationen stabil sein; Racemisierungen und Epimerisierungen müssen unterdrückt werden. Schutzgruppen sollen wieder unter milden Bedingungen selektiv und idealerweise mit hoher Ausbeute abspaltbar sein. Die Wahl einer geeigneten Schutzgruppe, die Bedingungen zur Umsetzung (Lösungsmittel, Temperatur, Dauer, etc.) aber auch die Möglichkeiten eine Schutzgruppe wieder zu entfernen sind im Stand der Technik bekannt (z.B. Philip Kocienski, Protecting Groups, 3rd ed. 2004, THIEME, Stuttgart, ISBN: 3131370033).

Unter einem "organischen Lösungsmittel" wird im Rahmen der Erfindung ein organsicher, niedermolekularer Stoff verstanden, der andere organische Stoffe auf physikalischem Wege zur Lösung bringen kann. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Adsorption in der Originalgestalt wieder gewonnen werden können. Aus verschiedenen Gründen können nicht nur die reinen Lösungsmittel, sondern Gemische, die die Lösungseigenschaften vereinigen verwendet werden. Beispielsweise seien genannt:
- Alkohole, bevorzugt Methanol, Ethanol, Propanol, Butanol, Octanol, Cyclohexanol;
- Glykole, bevorzugt Ethylenglykol, Diethylenglykol;
- Ether / Glykolether, bevorzugt Diethylether, tert-Butylmethylether, Dibutylether, Anisol, 1,4-Dioxan, Tetrahydrofuran, Mono-, Di-, Tri-, Polyethylenglykolether;
- Ketone, bevorzugt Aceton, Butanon, Cyclohexanon;
- Ester, bevorzugt Essigsäureester, Glykolester;
- Amide u.a. Stickstoff-Verbindungen, bevorzugt N,N-Dimethylformamid, Pyridin, N-Methylpyrrolidon, Acetonitril;
- Schwefel-Verbindungen, bevorzugt Schwefelkohlenstoff, Dimethylsulfoxid, Sulfolan;
- Nitro-Verbindungen bevorzugt Nitrobenzol;
- Halogenkohlenwasserstoffe, bevorzugt Dichlormethan, Chloroform, Tetrachlormethan, Tri-, Tetrachlorethen, 1,2-Dichlorethan, Chlorfluorkohlenstoffe;
- aliphatische oder alicyclische Kohlenwasserstoffe, bevorzugt Benzine, Petrolether, Cyclohexan, Methylcyclohexan, Decalin, Terpen-L.; oder
- aromatische Kohlenwasserstoffe, bevorzugt Benzol, Toluol, o-Xylol, m-Xylol, p-Xylol;
oder entsprechende Gemische davon.

Die Bezeichnung diastereomerenrein beschreibt im Rahmen der vorliegenden Erfindung Verbindungen der Formel (I), die in einer Diastereomerenreinheit von wenigstens 85%de, bevorzugt von wenigstens 90%de, besonders bevorzugt von > 95% de vorliegen. Die Bezeichnung de (diastereomeric excess) ist im Stand der Technik bekannt und beschreibt den optischen Reinheitsgrad diastereomerer Verbindungen.

Die Bezeichnung enantiomerenrein beschreibt im Rahmen der vorliegenden Erfindung Verbindungen der Formel (I), die in einer Enantiomerenreinheit von wenigstens 85%ee, bevorzugt von wenigstens 90%ee, besonders bevorzugt von > 95%ee vorliegen. Die Bezeichnung ee (enantiomeric excess) ist im Stand der Technik bekannt und beschreibt den optischen Reinheitsgrad chiraler Verbindungen.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatomen, besonders bevorzugt Alkylgruppen mit 1 bis 2 Kohlenstoffatomen. Beispielsweise werden hierfür genannt: Methyl, Ethyl, *n*-Propyl, *iso*-Propyl, *n*-Butyl, *iso*-Butyl, sec-Butyl, *tert*-Butyl, *n-*Pentyl, *iso*-Pentyl, *neo*-Pentyl oder Hexyl. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso-*Propyl, Butyl umfasst *iso*-Butyl, *sec*-Butyl und *tert*-Butyl etc.

Unter dem Begriff "C₁₋₃-Alkylen" (auch soweit sie Bestandteil anderer Reste sind) werden verzweigte und unverzweigte Alkylengruppen mit 1 bis 3 Kohlenstoffatomen verstanden. Bevorzugt sind Alkylengruppen mit 1 bis 2 Kohlenstoffatomen. Beispielsweise werden hierfür genannt: Methylen, Ethylen, Propylen, 1-Methylethylen, Butylen, 1-Methylpropylen, 1,1-Dimethylethylen und 1,2-Dimethylethylen. Sofern nicht anders beschrieben, umfasst die Definition Alkylen alle denkbaren isomeren Formen der jeweiligen Reste gleicher Kohlenstoffanzahl. So umfasst beispielsweise Propylen auch 1-Methylethylen.

Unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert-*Butyl, Hydroxy und Fluor.

Unter dem Begriff "Aryl" (auch soweit sie Bestandteil anderer Reste sind) werden aromatische Ringsysteme mit 6, 10 oder 14 Kohlenstoffatomen verstanden. Beispielsweise werden hierfür genannt: Phenyl, Naphthyl, Anthracenyl oder Phenanthrenyl, bevorzugter Arylrest ist Phenyl. Soweit nicht anders beschrieben, können die Aromaten substituiert sein mit einem oder mehreren Resten **R⁵**.

Insbesondere bevorzugt ist unter dem Begriff "Aryl" jeweils eine Phenylgruppe zu verstehen, die durch **R⁵** mono- oder disubstituiert ist, wobei die Substituenten **R⁵** gleich oder verschieden sein können und
- **R⁵**: Wasserstoff, oder einen Rest, ausgewählt aus der Gruppe bestehend aus F, Cl, Br, I, OH, CN, C₁₋₃-Alkyl-, C₁₋₃-Alkyl-O-, CHF₂, CF₃, -O-CHF₂ und -O-CF₃, bedeutet.

Unter dem Begriff "Heteroaryl" werden 5-10-gliedrige mono- oder bicyclische Heteroarylringe, in denen bis zu drei C-Atome durch ein oder mehrere Heteroatome ausgewählt aus der Gruppe Sauerstoff, Stickstoff und Schwefel ersetzt sein können bezeichnet, wobei diese so viele konjugierte Doppelbindungen enthalten, dass ein aromatisches System gebildet wird. Jeder der vorstehend genannten Heterocyclen kann gegebenenfalls ferner an einen Benzolring anelliert sein. Die Heteroarylringe können, soweit nicht anders beschrieben, beispielsweise einen oder mehrere Substituenten tragen.

Der Ring kann über ein Kohlenstoffatom oder falls vorhanden über ein Stickstoffatom mit dem Molekül verknüpft sein. Als Beispiele für fünf- oder sechsgliedrige heterocyclische Aromaten, werden genannt:

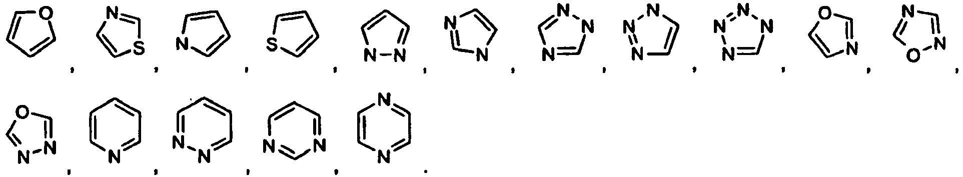

Als Beispiel für 5-10-gliedrige bicyclische Heteroarylringe werden genannt Pyrrolizin, Indol, Indolizin, Isoindol, Indazol, Purin, Chinolin, Isochinolin, Chinoxalin, Benzimidazol, Benzofuran, Benzothiophen, Benzothiazol, Benzoisothiazol, Pyridopyrimidin, Pteridin, Pyrimidopyrimidin. Insbesondere bevorzugt ist unter dem Begriff "Heteroaryl"
eine Pyridinyl-, Pyridazinyl-, Pyrimidinyl- oder Pyrazinyl-gruppe zu verstehen, wobei diese jeweils durch den Rest **R⁵** mono- oder disubstituiert sind, wobei die Substituenten **R⁵** gleich oder verschieden sein können und **R⁵** wie vorstehend erwähnt definiert ist.

"Halogen" steht im Rahmen der vorliegenden Erfindung für Fluor, Chlor, Brom oder Iod. Sofern nicht gegenteilig angegeben, gelten Fluor, Chlor und Brom als bevorzugte Halogene.

Der Substituent **R^{a}** kann eine Phenyl- oder 1-Phenylethyl-Gruppe, vorzugsweise eine PhenylGruppe, in denen der Phenylkern jeweils durch die Reste **R¹** bis **R³** substituiert ist, bedeuten. Insbesondere bevorzugt bedeutet der Substituent **R^{a}** einen Rest, ausgewählt aus der Gruppe bestehend aus 3-Chlor-2-fluor-phenyl-, 3-Chlor-4-fluor-phenyl-, 5-Chlor-2-fluor-phenyl-, 2-Fluor-3-methyl-phenyl-, 2-Fluor-5-methyl-phenyl-, 4-Fluor-3-methyl-phenyl- und 3-Chlor-2-methylphenyl-. Ganz besonders bevorzugt bedeutet der Substituent **R^{a}** eine 3-Chlor-2-fluor-phenylGruppe.

Der Substituent **R^{b}** kann Wasserstoff, oder einen Rest, gegebenenfalls substituiert, ausgewählt aus der Gruppe bestehend aus C₁₋₆-Alkyl, C₃₋₆-Cycloalkyl- und C₃₋₆-Cycloalkyl-C₁₋₃-alkyl, vorzugsweise Wasserstoff und C₁₋₃-Alkyl, insbesondere bevorzugt Wasserstoff und Methyl, bedeuten.

Der Substituent **R^{c}** kann Wasserstoff, oder einen Rest, gegebenenfalls substituiert, ausgewählt aus der Gruppe bestehend aus C₁₋₆-Alkyl, C₁₋₆-Cycloalkyl-, C₃₋₆-Cycloalkyl-C₁₋₃-alkyl, C₁₋₆-Alkyl-CO-, C₃₋₆-Cycloalkyl-CO-, C₃₋₆-Cycloalkyl-C₁₋₃-alkyl-CO-, C₁₋₆-Alkyl-SO₂-, C₃₋₆-Cycloalkyl-SO₂-, C₃₋₆Cycloalkyl-C₁₋₃-alkyl-SO₂-, Phenyl-CO- und Phenyl-SO₂-, vorzugsweise Wasserstoff und C₁-₃-Alkyl, insbesondere bevorzugt Wasserstoff und Methyl, bedeuten.

Der Substituent **R^{d}** kann Wasserstoff oder
einen Rest ausgewählt aus der Gruppe bestehend aus F, Cl, Br, I, OH, C₁₋₄-Alkyl, C₁₋₄-O-Alkyl-O-, mit 1 bis 3 Fluoratomen substituiertes C₁₋₂-Alkyl-O-, C₃₋₇-Cycloalkyl-O-, C₃₋₇-Cycloalkyl-C₁₋₄-alkyl-O-, Tetrahydrofuran-3-yl-O-, Tetrahydropyran-3-yl-O-, Tetrahydropyran-4-yl-O-, Tetrahydrofuranyl-C₁₋₄-alkyl-O- und Tetrahydropyranyl-C₁₋₄-alkyl-O-,
oder
R⁴-C₁₋₄-alkyl-, wobei die Verknüpfung der Reste **R⁴** über jedes C-Atom des Alkylrestes erfolgen kann,
oder
R⁴-C₂₋₄-alkyl-O-, wobei der Rest **R⁴** durch mindestens 2 C-Atome von dem Sauerstoffatom getrennt ist,
oder
einen Rest ausgewählt aus der Gruppe bestehend aus Pyrrolidin-2-yl-C₁₋₄-alkyl-O-, Pyrrolidin-3-yl-C₁₋₄-alkyl-O-, Piperidin-2-yl-C₁₋₄-alkyl-O-, Piperidin-3-yl-C₁₋₄-alkyl-O-, Piperidin-4-yl-C₁₋₄-alkyl-O-, Azepan-2-yl-C₁₋₄-alkyl-O-, Azepan-3-yl-C₁₋₄-alkyl-O-, Azepan-4-yl-C₁₋₄-alkyl-O-, Morpholin-2-yl-C₁₋₄-alkyl-O-, Morpholin-3-yl-C₁₋₄-alkyl-O-, 1-(C₁₋₄-Alkyl)-pyrrolidin-2-yl-C₁₋₄-alkyl-O-, 1-(C₁₋₃-Alkyl)-pyrrolidin-3-yl-C₁₋₄-alkyl-O-, 1-(C₁₋₃-Alkyl)-piperidin-2-yl-C₁₋₄-alkyl-O-, 1-(C₁₋₃-Alkyl)-piperidin-3-yl-C₁₋₄-alkyl-O-, 1-(C₁₋₃-Alkyl)-piperidin-4-yl-C₁₋₄-alkyl-O-, 1-(C₁₋₃-Alkyl)-azepan-2-yl-C₁₋₄-alkyl-O-, 1-(C₁₋₃-Alkyl)-azepan-3-yl-C₁₋₄-alkyl-O-, 1-(C₁₋₃-Alkyl)-azepan-4-yl-C₁₋₄-alkyl-O-, 4-(C₁₋₃-Alkyl)-morpholin-2-yl-C₁₋₄-alkyl-O- und 4-(C₁₋₃-Alkyl)-morpholin-3-yl-C₁₋₄-alkyl-O-,
vorzugsweise C₁₋₃-Alkyl-O-, insbesondere bevorzugt CH₃-O- ,
wobei die bei der Definition des Restes **R^{d}** vorstehend genannten Pyrrolidinyl-, Piperidinyl-, Azepan-1-yl-, Piperazinyl-, 1,4-Diazepan-1-yl-, Morpholinyl- und 1,4-Oxazepan-4-yl-Gruppen jeweils zusätzlich durch eine oder zwei C₁₋₃-Alkylgruppen substituiert sein können,
und
wobei die vorstehend genannten Phenylgruppen durch Reste **R⁵** mono- oder di- substituiert sind,
bedeuten.

Der Substituent **R¹** kann Wasserstoff oder einen Rest ausgewählt aus der Gruppe bestehend aus F, Cl, Br, I, OCH₂F, OCHF₂, OCF₃, CH₂F, CHF₂, CF₃, CN, NO₂, NH₂ und OH,
oder
einen Rest ausgewählt aus der Gruppe bestehend aus
C₁₋₄-Alkyl C₁₋₄-Alkyl-O-, C₂₋₃-Alkenyl, C₂₋₃-Alkinyl,
Phenyl, Phenyl-O-, Phenyl-C₁₋₃-alkyl-, Phenyl-C₁₋₃-alkyl-O-, Heteroaryl, Heteroaryl-O-, Heteroaryl-C₁₋₃-alkyl- und Heteroaryl-C₁₋₃-alkyl-O,
wobei die vorstehend genannten Phenylgruppen durch Reste **R⁵**, mono- oder disubstituiert sind,
vorzugsweise Wasserstoff, Fluor, Chlor, Brom oder Methyl, insbesondere bevorzugt Wasserstoff, Fluor, Chlor oder Methyl bedeuten.

Der Substituent **R²** kann Wasserstoff oder
einen Rest ausgewählt aus der Gruppe bestehend aus F, Cl, Br, I, OCH₂F, OCHF₂, OCF₃, CH₂F, CHF₂, CF₃, CN, NO₂, NH₂ und OH,
oder
einen Rest ausgewählt aus der Gruppe bestehend aus
C₁₋₄-Alkyl, C₁₋₄-Alkyl-O-, C₂₋₃-Alkenyl, C₂₋₃-Alkinyl,
Phenyl, Phenyl-O-, Phenyl-C₁₋₃-alkyl-, Phenyl-C₁₋₃-alkyl-O-, Heteroaryl, Heteroaryl-O-, Heteroaryl-C₁₋₃-alkyl- und Heteroaryl-C₁₋₃-alkyl-O, wobei die vorstehend genannten
Phenylgruppen durch Reste **R⁵** mono- oder di- substituiert sind,
vorzugsweise Wasserstoff, Fluor, Chlor oder Methyl, insbesondere bevorzugt Wasserstoff, Fluor oder Chlor bedeuten.

Der Substituent **R³** kann Wasserstoff, oder
einen Rest ausgewählt aus der Gruppe bestehend aus F, Cl, Br und CH₃. vorzugsweise Wasserstoff bedeuten.

Der Substituent **R⁴** kann einen Rest, gleich oder verschieden, ausgewählt aus der Gruppe bestehend aus

OH, C₁₋₃-Alkyl-O-, C₃₋₆-Cycloalkyl-O-, NH₂, C₁₋₃-Alkyl-NH-, (C₁₋₃-Alkyl)₂N-, (2-Methoxyethyl)₂N-, Pyrrolidin-1-yl-, Piperidin-1-yl-, Azepan-1-yl-, Morpholin-4-yl-, 1,4-Oxazepan-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo[3.2.1]oct-3-yl-, Piperazin-1-yl-, 4-(C₁₋₃-Alkyl)-piperazin-1-yl-, 1,4-Diazepan-1-yl-, 4-(C₁₋₃-Alkyl)-1,4-diazepan-1-yl, HCO-NH-, C₁₋₄-Alkyl-CO-NH-, C₁₋₃-Alkyl-O-C₁₋₃-alkyl-CO-NH-, C₁₋₄-Alkyl-O-CO-NH-, H₂NCONH-, C₁₋₃-Alkyl-NH-CO-NH-, (C₁₋₃-Alkyl)₂N-CONH-, Pyrrolidin-1-yl-CO-NH-, Piperidin-1-yl-CO-NH -, Piperazin-1-yl-CO-NH-, 4-(C₁₋₃-Alkyl)-piperazin-1-yl-CO-NH-, Morpholin-4-yl-CO-NH- und C₁₋₄-Alkyl-SO₂-NH- bedeuten.

Der Substituent **R⁵** kann Wasserstoff, oder
einen Rest, gleich oder verschieden, ausgewählt aus der Gruppe bestehend aus F, Cl, Br, I, OH, CN, C₁₋₃-Alkyl-, C₁₋₃-Alkyl-O-, CHF₂. CF₃, -O-CHF₂ und -O-CF₃, bedeuten.

**A** kann -CO- oder -C₁-C₃-alkylen-, vorzugsweise -CH₂CH₂-,
wobei der -C₁-C₃-alkylen- Rest durch einen Rest **R⁶** 1-, 2-, 3- oder 4-fach, vorzugsweise 1- oder 2- fach, substituiert sein kann

Der Substituent **R⁶** kann gleich oder verschieden, Wasserstoff, oder
einen Rest ausgewählt aus der Gruppe bestehend aus OH, C₁-C₄-Alkyl und -O-C₁-C₄-Alkyl, vorzugsweise Methyl,
bedeuten.

Eine besonders bevorzugte Bedeutung für A ist -CH₂CH₂-.

### Herstellungsverfahren

Zur Herstellung von Verbindungen der allgemeinen Formel (I) sind beispielsweise folgenden Verfahren geeignet:
a) Umsetzung einer Verbindung der allgemeinen Formel 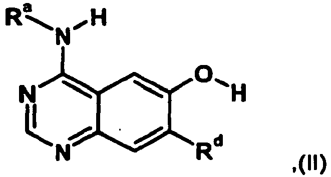 in der
   **R^{a}** und **R^{d}** wie eingangs erwähnt definiert sind, mit einer Verbindung der allgemeinen Formel 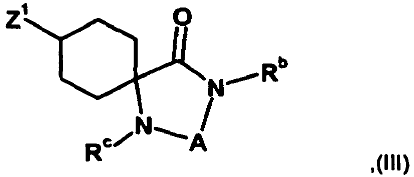 in der
   **R^{b}, R^{c}** und **A** wie eingangs erwähnt definiert sind und Z¹ eine Austrittsgruppe wie ein Halogenatom, z.B. ein Chlor- Brom- oder Iodatom, eine Sulfonyloxygruppe wie eine Methansulfonyloxy- oder p-Toluolsulfonyloxygruppe oder eine Hydroxygruppe darstellt.

Mit einer Verbindung der allgemeinen Formel (III), in der Z¹ ein Halogenatom oder eine Sulfonyloxygruppe darstellt, erfolgt die Umsetzung zweckmäßigerweise in einem Lösungsmittel wie Ethanol, Isopropanol, Acetonitril, Toluol, Tetrahydrofuran, 1,4-Dioxan, N,N-Dimethylformamid, Dimethylsulfoxid oder N-Methylpyrrolidinon, vorzugsweise in Gegenwart einer Base wie Kaliumcarbonat, Cesiumcarbonat, Kaliumhydroxid, Kalium-tert-butylat, Natriumhydrid oder N-Ethyl-diisopropylamin, bei Temperaturen im Bereich von 20°C bis 160°C, beispielsweise bei Temperaturen im Bereich von 60°C bis 140°C.

Mit einer Verbindung der allgemeinen Formel III, in der Z¹ eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, 1,4-Dioxan, Toluol oder Ethylenglycoldiethylether bei Temperaturen zwischen -50 und 150°C, vorzugsweise jedoch bei Temperaturen zwischen -20 und 80°C, durchgeführt.
b) Zur Herstellung von Verbindungen der allgemeinen Formel I, in der **R^{b}** und **R^{c}** jeweils ein Wasserstoffatom und A eine -CO- Gruppe bedeuten, Umsetzung einer Verbindung der allgemeinen Formel 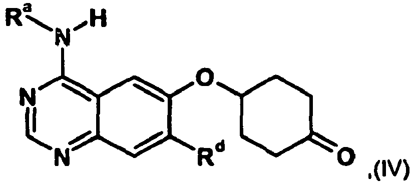 in der
   **R^{a}** und **R^{d}** wie eingangs erwähnt definiert sind, mit einem Alkalimetallcyanid und Ammoniumcarbonat.

Die Reaktion wird beispielsweise in einem Lösemittel oder Lösemittelgemisch wie Methanol, Ethanol, Ethanol/Wasser oder Isopropanol bei Temperaturen zwischen Raumtemperatur und 120°C durchgeführt. Weitere Hinweise zur Synthese von Hydantoinen finden sich beispielsweise in der folgenden Publikation:

Meusel, M.; Guetschow, M., Organic Preparations and Procedures International (2004), 36(5), 391-443.
c) Umsetzung einer Verbindung der allgemeinen Formel (V) 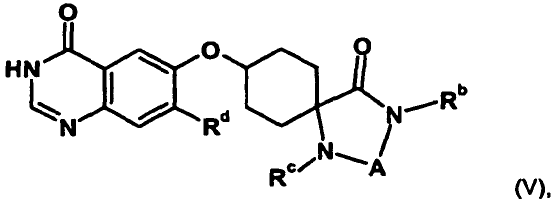
in der R**^{b}, R^{c}, R^{d}** und **A** wie eingangs erwähnt definiert sind, mit einem Halogenierungsmittel, beispielsweise einem Säurehalogenid wie Thionylchlorid, Thionylbromid, Phosphortrichlorid, Phosphorpentachlorid oder Phosphoroxychlorid zu einer Zwischenverbindung der allgemeinen Formel (VI), 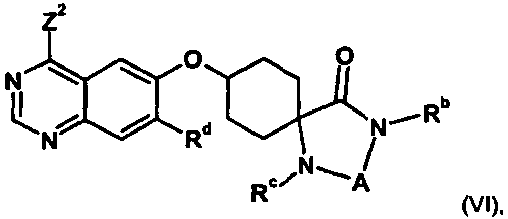 in der **R^{b}, R^{c}, R^{d}** und **A** wie eingangs erwähnt definiert sind und Z² ein Halogenatom wie ein Chlor- oder Bromatom darstellt,
und anschließender Umsetzung mit einer Verbindung der allgemeinen Formel (VII),
**R^{a}**-NH₂ (VII), in der **R^{a}** wie eingangs erwähnt definiert ist, oder dessen Salzen.

Die Umsetzung mit dem Halogenierungsmittel wird gegebenenfalls in einem Lösungsmittel wie Methylenchlorid, Chloroform, Acetonitril oder Toluol und gegebenfalls in Gegenwart einer Base wie N,N-Diethylanilin, Pyridin, Triethylamin oder N-Ethyl-diisopropylamin bei Temperaturen im Bereich von 20°C bis 160°C, vorzugsweise von 40°C bis 120°C durchgeführt. Vorzugsweise wird die Reaktion jedoch mit Thionylchlorid und katalytischen Mengen an N,N-Dimethylformamid bei der Siedetemperatur des Reaktionsgemisches oder aber mit Phosphoroxychlorid in Acetonitril in Gegenwart von Triethylamin bei der Siedetemperatur des Reaktionsgemisches durchgeführt.

Die Umsetzung der Verbindung der allgemeinen Formel (VI) mit der Verbindung der allgemeinen Formel (VII) oder deren Salzen erfolgt zweckmäßigerweise in einem Lösungsmittel wie Ethanol, Isopropanol, Acetonitril, 1,4-Dioxan oder N,N-Dimethylformamid, gegebenfalls in Gegenwart einer Base wie Kaliumcarbonat, Triethylamin oder N-Ethyl-diisopropylamin, bei Temperaturen im Bereich von 20°C und 160°C, vorzugsweise von 60°C bis 120°C. Vorzugsweise wird die Reaktion jedoch in Isopropanol bei der Siedetemperatur des Reaktionsgemisches durchgeführt.

Die Umsetzung einer Verbindung der allgemeinen Formel (V) zu einer Verbindung der allgemeinen Formel (I) kann auch als Eintopfreaktion, beispielsweise in Acetonitril in Gegenwart von Triethylamin, durchgeführt werden.
d) Zur Herstellung von Verbindungen der allgemeinen Formel I, in der R^{d} eine der eingangs
erwähnten, gegebenenfalls substituierten Alkyloxygruppen darstellt:

Umsetzung einer Verbindung der allgemeinen Formel 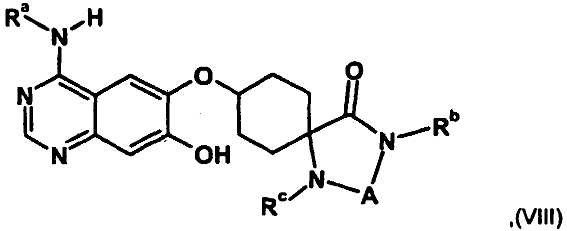 in der **R^{a}, R^{b}, R^{c}** und **A** wie eingangs erwähnt definiert sind, mit einer Verbindung der allgemeinen Formel

Z³ - **R^{d'}** (IX)

in der **R^{a'}** einen Rest bedeutet ausgewählt aus der Gruppe bestehend aus C₁₋₄-Alkyl, mit 1 bis 3 Fluoratomen substituiertes C₁₋₂-Alkyl-, C₃-₇-Cycloalkyl-, C₃-₇-Cycloalkyl-C₁₋₄-alkyl-, Tetrahydrofuran-3-yl-, Tetrahydropyran-3-yl-, Tetrahydropyran-4-yl-, Tetrahydrofuranyl-C₁₋₄-alkyl- und Tetrahydropyranyl-C₁₋₄-alkyl-,
oder
R⁴-C₂₋₄-alkyl-, wobei der Rest **R⁴** durch mindestens 2 C-Atome von **Z³** getrennt ist,
oder
einen Rest bedeutet ausgewählt aus der Gruppe bestehend aus Pyrrolidin-2-yl-C₁₋₄-alkyl-, Pyrrolidin-3-yl-C₁₋₄-alkyl-, Piperidin-2-yl-C₁₋₄-alkyl-, Piperidin-3-yl-C₁₋₄-alkyl-, Piperidin-4-yl-C₁₋₄-alkyl-, Azepan-2-yl-C₁₋₄-alkyl-, Azepan-3-yl-C₁₋₄-alkyl-, Azepan-4-yl-C₁₋₄-alkyl-, Morpholin-2-yl-C₁₋₄-alkyl-, Morpholin-3-yl-C₁₋₄-alkyl-, 1-(C₁₋₃-Alkyl)-pyrrolidin-2-yl-C₁₋₄-alkyl-, 1-(C₁₋₃-Alkylrpyrrolidin-3-yl-C₁₋₄-alkyl-, 1-(C₁₋₃-Alkyl)-piperidin-2-yl-C₁₋₄-alkyl-, 1-(C₁₋₃-Alkyl)-piperidin-3-yl-C₁₋₄-alkyl-, 1-(C₁₋₃-Alkyl)-piperidin-4-yl-C₁₋₄-alkyl-, 1-(C₁₋₃-Alkyl)-azepan-2-yl-C₁₋₄-alkyl-, 1-(C₁₋₃-Alkyl)-azepan-3-yl-C₁₋₄-alkyl-, 1-(C₁₋₃-Alkyl)-azepan-4-yl-C₁₋₄-alkyl-, 4-(C₁₋₃-Alkyl)-morpholin-2-yl-C₁₋₄-alkyl-, 4-(C₁₋₃-Alkyl)-morpholin-3-yl-C₁₋₄-alkyl-,
und
Z³ eine Austrittsgruppe wie ein Halogenatom, eine Alkylsulfonyloxy-, Arylsulfonyloxy- oder eine Hydroxygruppe darstellt.

Handelt es sich bei der Austrittsgruppe um ein Halogenatom wie ein Chlor-, Brom- oder lodatom oder um eine Alkylsulfonyloxy- oder Arylsulfonyloxygruppe wie die Methansulfonyloxy- oder p-Toluolsulfonyloxygruppe, wird die Reaktion vorzugsweise in Gegenwart einer organischen oder anorganischen Base wie Kaliumcarbonat, Cesiumcarbonat, Kaliumhydroxid, Natriumhydrid oder N-Ethyl-diisopropylamin durchgeführt. Handelt es sich bei der Austrittsgruppe um eine Hydroxygruppe, so wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester durchgeführt.
e) Zur Herstellung von Verbindungen der allgemeinen Formel I, in der R^{d} eine R^{4'}-C₂₋₄-alkyl-O- Gruppe darstellt, wobei der Rest R^{4'} durch mindestens 2 C-Atome von dem Sauerstoffatom getrennt ist, und R^{4'} einen Rest bedeutet ausgewählt aus der Gruppe bestehend aus NH₂, C₁₋₃-Alkyl-NH-, (C₁₋₃-Alkyl)₂N-, (2-Methoxyethyl)₂N-, Pyrrolidin-1-yl-, Piperidin-1-yl-, Azepan-1-yl-, Morpholin-4-yl-, 1,4-Oxazepan-4-yl-, 2-Oxa-5-aza-bicyclo[2.2.1]hept-5-yl-, 3-Oxa 8-aza-bicyclo[3.2.1]oct-8-yl-, 8-0xa-3-aza-bicyclo[3.2.1]oct-3-yl-, Piperazin-1-yl-, 4-(C₁₋₃-Alkyl) piperazin-1-yl-, 1,4-Diazepan-1-yl-, 4-(C₁₋₃-Alkyl)-1,4-diazepan-1-yl:
   Umsetzung einer Verbindung der allgemeinen Formel 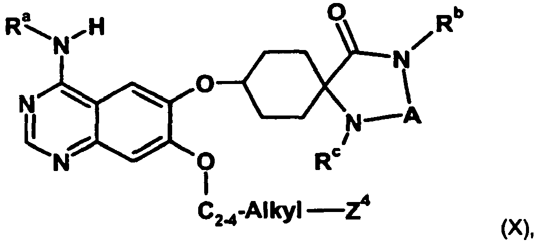 in der **R^{a}, R^{b}, R^{c}** und A wie eingangs erwähnt definiert sind und Z⁴ eine Austrittsgruppe wie ein Halogenatom, z.B. ein Chlor- Brom- oder lodatom oder eine Sulfonyloxygruppe wie eine Methansulfonyloxy- oder p-Toluolsulfonyloxygruppe darstellt, mit

   H - R^{4'} , (XI)

   wobei R^{4'} wie vorstehend erwähnt definiert ist.
   f) Zur Herstellung von Verbindungen der allgemeinen Formel I, in der **R^{b}** ein Wasserstoffatom bedeutet:

Abspaltung eines Schutzrestes von einer Verbindung der allgemeinen Formel 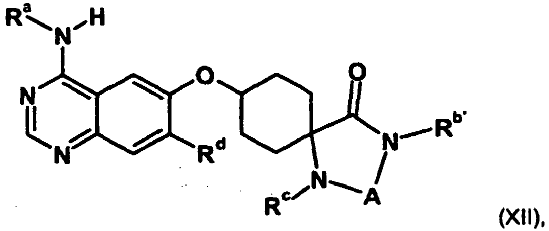 in der **R^{a}, R^{c}, R^{d}** und A wie eingangs erwähnt definiert sind und R^{b'} eine Schutzgruppe bedeutet, beispielsweise eine gegebenenfalls substituierte Benzylgruppe, eine tert.-Butylgruppe oder eine 2-(Trimethylsilyl)ethyl-Gruppe.

Die Abspaltung einer gegebenenfalls substituierten Benzyl-Gruppe erfolgt beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt vorzugsweise in Trifluoressigsäure in Gegenwart von Anisol, Thioanisol, Pentamethylbenzol oder Triethylsilan.

Die Abspaltung eines gegebenenfalls substituierten Benzyl-Restes oder eines tert.-Butyl-Restes kann beispielsweise auch durch Behandlung mit einer Säure wie Trifluoressigsäure, Salzsäure oder Bromwasserstoffsäure, gegebenenfalls unter Verwendung eines Lösungsmittels wie 1,4-Dioxan, Isopropanol, Methylenchlorid oder Toluol, gegebenenfalls in Gegenwart von Anisol, Thioanisol, Pentamethylbenzol oder Triethylsilan erfolgen.

Die Spaltung einer 2-(Trimethylsilyl)ethyl-Gruppe erfolgt beispielsweise durch Behandlung mit Fluoriden wie Tetrabutylammoniumfluorid gegebenenfalls unter Verwendung eines Lösungsmittels wie Tetrahydrofuran oder 1,4-Dioxan.

Weitere geeignete Schutzgruppen und Möglichkeiten zu ihrer Einführung und Abspaltung sind beispielsweise in "Protective Groups in Organic Synthesis" von Theodora W. Greene und Peter G. M. Wuts , Wiley-VCH, oder Philip Kocienski, Protecting Groups, 3rd ed. 2004, THIEME beschrieben.
g) Zur Herstellung von Verbindungen der allgemeinen Formel I, in der **R^{c}** ein Wasserstoffatom bedeutet:

Abspaltung eines Schutzrestes von einer Verbindung der allgemeinen Formel 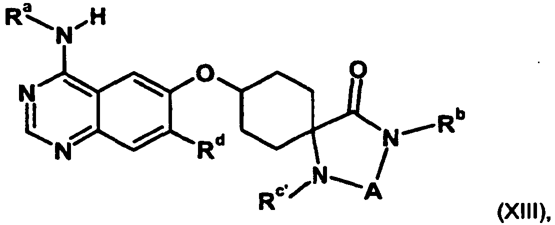 in der **R^{a}, R^{b}, R^{d}** und A wie eingangs erwähnt definiert sind und R^{c'} eine Schutzgruppe bedeutet, beispielsweise eine gegebenenfalls substituierte Benzylgruppe oder eine Formyl-, Acetyl-, Trifluoracetyl-, Methoxycarbonyl-, Ethoxycarbonyl-, tert.-Butoxycarbonyl- oder Benzyloxycarbonyl-Gruppe.

Die Abspaltung des Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder 1,4-Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Iodtrimethylsilan, bei Temperaturen zwischen 0 und 120°C, vorzugsweise bei Temperaturen zwischen 10 und 100°C.

Die Abspaltung einer gegebenenfalls substituierten Benzyl-Gruppe, oder eines Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar.

Die Abspaltung eines tert.-Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, 1,4-Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Weitere geeignete Schutzgruppen und Möglichkeiten zu ihrer Einführung und Abspaltung sind beispielsweise in "Protective Groups in Organic Synthesis" von Theodora W. Greene und Peter G. M. Wuts, Wiley-VCH, oder Philip Kocienski, Protecting Groups, 3rd ed. 2004, THIEME beschrieben.
h) Zur Herstellung von Verbindungen der allgemeinen Formel I, in der A eine -C₂-C₃-AlkylenGruppe bedeutet:

Cyclisierung einer Verbindung der allgemeinen Formel 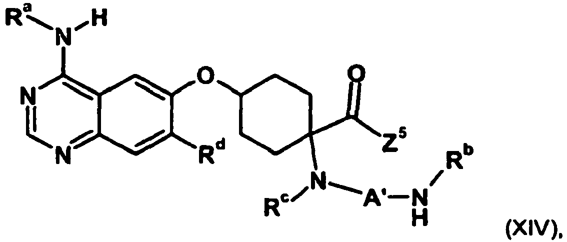 in der **R^{a}, R^{b}, R^{c}** und **R^{d}** wie eingangs erwähnt definiert sind, A' eine -C₂-C₃-Alkylen-Gruppe und Z⁵ eine Austrittsgruppe wie ein Halogenatom, eine Hydroxy- oder Alkyloxygruppe bedeutet.

Handelt es sich bei der Austrittsgruppe um eine Hydroxygruppe, so wird die Umsetzung in Gegenwart eines wasserentziehenden Mittels wie N,N'-Carbonyldiimidazol, N,N'-Dicyclohexylcarbodiimid, O-(Benzotriazol-1-yl)-N,N,N'N'-tetramethyluronium-tetrafluoroborat (TBTU) oder O-(7-Azabenzotriazol-1-yl)-N,N,N',N'-tetramethyluronium-hexafluorophosphat (HATU), zweckmäßigerweise in einem Lösungsmittel wie Methylenchlorid, N,N-Dimethylformamid, Acetonitril, Tetrahydrofuran, 1,4-Dioxan oder Ethylenglycoldiethylether bei Temperaturen zwischen -50°C und 100°C, vorzugsweise jedoch bei Temperaturen zwischen -20°C und 60°C, durchgeführt.

Handelt es sich bei der Austrittsgruppe um ein Halogenatom, so wird die Umsetzung vorzugsweise in Gegenwart einer Base wie Triethylamin, Pyridin oder N-Ethyl-diisopropylamin zweckmäßigerweise in einem Lösungsmittel wie Methylenchlorid, N,N-Dimethylformamid, Acetonitril, Tetrahydrofuran, 1,4-Dioxan oder Ethylenglycoldiethylether bei Temperaturen zwischen -50°C und 100°C, vorzugsweise jedoch bei Temperaturen zwischen -20°C und 60°C, durchgeführt.

Handelt es sich bei der Austrittsgruppe um ein Alkyloxygruppe, so wird die Umsetzung gegebenenfalls in Gegenwart einer Base wie Kaliumcarbonat, Natriumhydroxid, Triethylamin oder N-Ethyl-diisopropylamin zweckmäßigerweise in einem Lösungsmittel wie Methanol, Ethanol, Isopropanol, Methylenchlorid, N,N-Dimethylformamid, Acetonitril, Tetrahydrofuran, 1,4-Dioxan oder Ethylenglycoldiethylether bei Temperaturen zwischen -50°C und 120°C, vorzugsweise jedoch bei Temperaturen zwischen 0°C und 80°C, durchgeführt.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden, wobei als Acylierungsmittel beispielsweise Carbonsäurehalogenide, Carbonsäureanhydride und Carbonsäuren mit Aktivierungsmitteln wie N,N'-Carbonyldümidazol, N,N'-Dicyclohexylcarbodiimid oder O-(Benzotriazol-1-yl)-N,N,N'N'-tetramethyluronium-tetrafluoroborat und als Sulfonylierungsmittel Sulfonylhalogenide in Frage kommen, und/oder
eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden und/oder
eine Verbindung der allgemeinen Formel I, die eine Alkoxycarbonylgruppe enthält, so kann diese durch Esterspaltung in eine Carbonsäure übergeführt werden und/oder
eine Verbindung der allgemeinen Formel I, die eine Alkoxycarbonylgruppe enthält, so kann diese durch Umsetzung mit einem Amin in ein Carbonsäureamid-Derivat übergeführt werden und/oder
eine Verbindung der allgemeinen Formel I, die eine Carboxy-Gruppe enthält, so kann diese durch Umsetzung mit einem Amin in ein Carbonsäureamid-Derivat übergeführt werden.

Bei den vorstehend beschriebenen Umsetzungen können gegebenenfalls vorhandene reaktive Gruppen wie Hydroxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropano/Wasser, Essigsäure/Wasser, Tetrahydrofuran/Wasser oder 1,4-Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von lodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol, Thioanisol, Pentamethylbenzol oder Triethylsilan.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, 1,4-Dioxan, Methanol, Isopropanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Weitere geeignete Schutzgruppen und Möglichkeiten zu ihrer Einführung und Abspaltung sind beispielsweise in "Protective Groups in Organic Synthesis" von Theodora W. Greene und Peter G. M. Wuts , Wiley-VCH, oder Philip Kocienski, Protecting Groups, 3rd ed. 2004, THIEME beschrieben.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D-und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren oder Basen übergeführt werden. Als Säuren kommen hierfür beispielsweise Salzsäure, Bromwasserstoffsäure, Schwefelsäure, Methansulfonsäure, Ethansulfonsäure, Benzolsulfonsäure, p-Toluolsulfonsäure, Phosphorsäure, Fumarsäure, Bernsteinsäure, Benzoesäure, Salicylsäure, Mandelsäure, Milchsäure, Malonsäure, Zitronensäure, L-Äpfelsäure, L-Weinsäure oder Maleinsäure in Betracht. Als Basen kommen hierfür beispielsweise Natronlauge, Kalilauge, Calciumhydroxid, Diethanolamin oder N-Methyl-D-glucamine in Betracht.

Die als Ausgangsstoffe verwendeten Verbindungen der allgemeinen Formeln II bis XIV sind teilweise literaturbekannt oder können nach an sich literaturbekannten Verfahren (siehe Beispiele I bis XII) oder den vorstehend beschriebenen Verfahren, gegebenenfalls unter zusätzlicher Einführung von Schutzresten erhalten werden.

Standardverfahren zur Herstellung der Ausgangsmaterialien sind beispielsweise in "March's Advanced Organic Chemistry" von Michael B. Smith and Jerry March, Wiley-VCH oder in "Science of Synthesis/Houben-Weyl", Thieme, beschrieben.

Beispielsweise können die Verbindungen der allgemeinen Formel (V) und (VI) wie folgt erhalten werden:

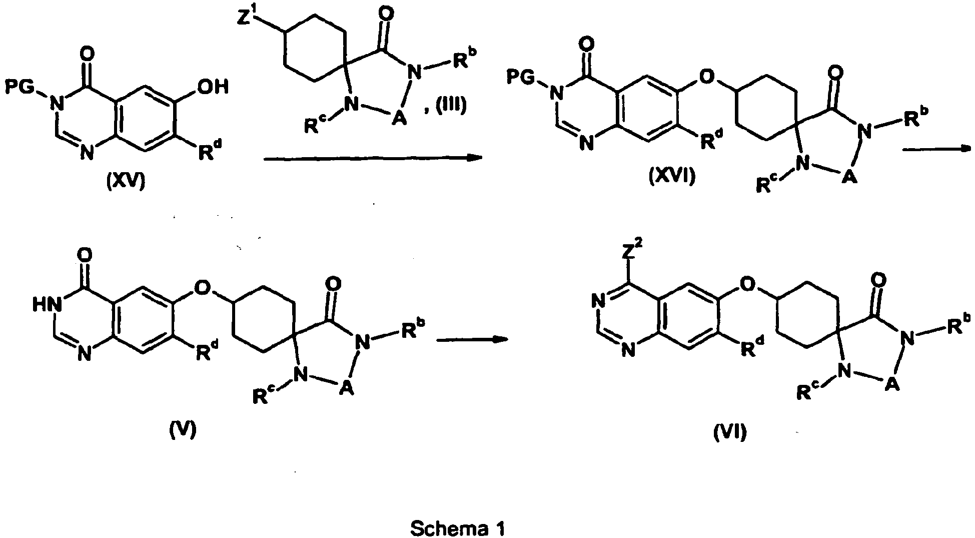

Ausgehend von einer Verbindung der allgemeinen Formel (XV), in der PG eine Schutzgruppe wie beispielsweise Benzyl, 4-Methoxybenzyl oder 2,4-Dimethoxybenzyl bedeutet, erfolgt die Umsetzung mit einer Verbindung der allgemeinen Formel (III) analog des vorstehend beschriebenen Verfahrens a) zu einer Verbindung der allgemeinen Formel (XVI). Die Verbindungen der allgemeinen Formel (XVI) sind literaturbekannt (siehe z. B. WO 2004/108664 oder WO 2007/003486) oder können nach an sich literaturbekannten Verfahren erhalten werden.

Die Abspaltung des Schutzrestes aus einer Verbindung der allgemeinen Formel (XVI) zu einer Verbindung der allgemeinen Formel (V) erfolgt, falls PG Benzyl bedeutet, beispielsweise mit Wasserstoff in Gegenwart eines Katalysators wie Palladium/Kohle (z. B. analog Beispiel XI). Die Abspaltung des Schutzrestes, falls PG 4-Methoxybenzyl oder 2,4-Dimethoxybenzyl bedeutet, kann auch oxidativ (z.B. mit Cer(IV)-ammoniumnitrat oder mit 2,3-Dichlor-5,6-dicyano-1,4-benzochinon) oder mit Säuren (z.B. mit Trifluoressigssäure in Gegenwart von Anisol, Thioanisol, Pentamethylbenzol oder Triethylsilan) erfolgen.

Eine Verbindung der allgemeinen Formel (V) kann dann, wie im vorstehenden Verfahren c) beschrieben, in eine Verbindung der allgemeinen Formel (VI) übergeführt werden. Die Bedeutungen für R**^{b}, R^{c}, R^{d}**, A, Z¹ und Z² in den Verbindungen des Schema 1 sind wie vorstehend erwähnt definiert.

Wie bereits eingangs erwähnt, weisen die erfindungsgemäßen Verbindungen der allgemeinen Formel (I) und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, dass die Signalübertragung an weiter abwärtsliegenden Komponenten blockiert wird.

Die nachfolgenden Beispiele sollen die vorliegende Erfindung näher erläutern ohne diese zu beschränken:

### Herstellung der Ausgangsverbindungen

### Beispiel I

### trans-1-(2-Amino-ethylamino)-4-[4-(3-chlor-2-fluor-phenylamino)-7-methoxy-chinazolin-6-yloxy]-cyclohexancarbonsäure-methylester

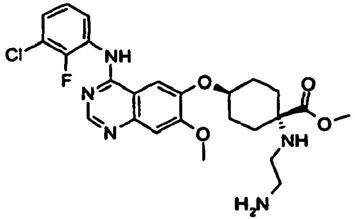

Zu 1.60 g trans-1-(2-tert.-Butoxycarbonylamino-ethylamino)-4-[4-(3-chlor-2-fluor-phenylamino)-7-methoxy-chinazolin-6-yloxy]-cyclohexancarbonsäure-methylester in 13 ml Methylenchlorid werden 3.30 ml Trifluoressigsäure gegeben. Das Reaktionsgemisch wird drei Stunden bei Raumtemperatur gerührt, dann wird nochmals 1 ml Trifluoressigsäure zugesetzt. Nach einer weiteren Stunde ist die Umsetzung vollständig und das Reaktionsgemisch wird eingeengt, in Methylenchlorid und etwas Methanol aufgenommen und mit 10%iger Kaliumcarbonat-Lösung alkalisch gestellt. Die organische Phase wird abgetrennt und die wässrige Phase mit Methylenchlorid extrahiert. Die vereinigten organischen Phasen werden über Magnesiumsulfat getrocknet und eingeengt.
Ausbeute: 1.30 g (97 % der Theorie)
Massenspektrum (ESI⁺): m/z = 518, 520 [M+H]⁺

Analog Beispiel I werden folgende Verbindungen erhalten:
(1) trans-1-Amino-4-[4-(3-chlor-2-fluor-phenylamino)-7-methoxy-chinazolin-6-yloxy]-cyclohexancarbonsäure-methylester 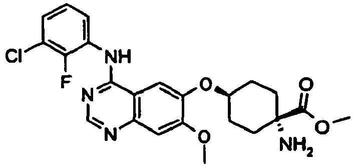 Massenspektrum (ESI⁺): m/z = 475, 477 [M+H]⁺
(2) trans-4-[4-(3-Chlor-2-fluor-phenylamino)-7-methoxy-chinazolin-6-yloxyl-1-(2-methylamino-ethylamino)-cyclohexancarbonsäure-methylester 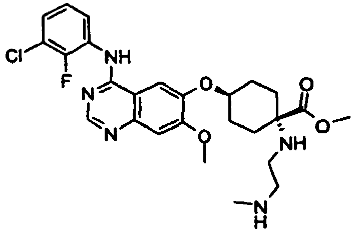 Massenspektrum (ESI⁺): m/z = 532, 534 [M+H]⁺
(3) cis-1-(2-Amino-ethylamino)-4-[4-(3-chlor-2-fluor-phenylamino)-7-methoxy-chinazolin-6-yloxy]-cyclohexancarbonsäure-methylester

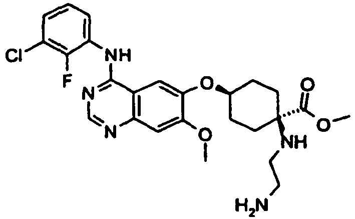

### Beispiel II

### trans-1-(2-tert.-Butoxycarbonylamino-ethylamino)-4-[4-(3-chlor-2-fluor-phenylamino)-7-methoxy-chinazolin-6-yloxyl-cyclohexancarbonsäure-methylester

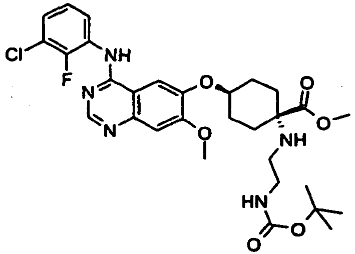

Zu 1.20 g trans-1-Amino-4-[4-(3-chlor-2-fluor-phenylamino)-7-methoxy-chinazolin-6-yloxy]-cyclohexancarbonsäure-methylester in 40 ml Tetrahydrofuran werden unter Argonatmosphäre 0.44 g N-tert.-Butoxycarbonyl-2-aminoacetaldehyd gegeben. Anschließend werden 0.18 ml Eisessig und 0.80 g Natriumtriacetoxyborhydrid zugegeben und das Reaktionsgemisch wird über Nacht bei Raumtemperatur gerührt. Da die Umsetzung noch nicht vollständig ist, werden noch zweimal je 90 mg N-tert.-Butoxycarbonyl-2-aminoacetaldehyd und je 200 mg Natriumtriacetoxyborhydrid zugesetzt. Nach einer weiteren Nacht bei Raumtemperatur ist die Umsetzung vollständig. Das Reaktionsgemisch wird mit Essigester verdünnt und mit Natronlauge versetzt. Die organische Phase wird abgetrennt und die wässrige Phase mit Essigester extrahiert. Die vereinigten organischen Phasen werden mit gesättigter Natriumchlorid-Lösung gewaschen, über Magnesiumsulfat getrocknet und eingeengt. Das Rohprodukt wird ohne weitere Reinigung weiter umgesetzt.
R_{f}-Wert: 0.35 (Kieselgel, Methylenchlorid/Methanol/konz. Ammoniak = 90:10:1) Massenspektrum (ESI⁺): m/z = 618, 620 [M+H]⁺

Analog Beispiel II werden folgende Verbindungen erhalten:
(1) trans-1-[2-(N-tert.-Butoxycarbonyl-N-methyl-amino)-ethylamino]-4-[4-(3-chlor-2-fluor-phenylamino)-7-methoxy-chinazolin-6-yloxy]-cyclohexancarbonsäure-methylester 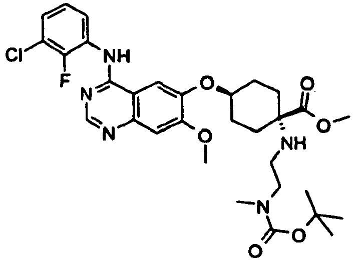 Massenspektrum (ESI⁺): m/z = 632, 634 [M+H]⁺
(2) cis-1-(2-tert.-Butoxycarbonylamino-ethylamino)-4-[4-(3-chlor-2-fluor-phenylamino)-7-methoxy-chinazolin-6-yloxy]-cyclohexancarbonsäure-methylester 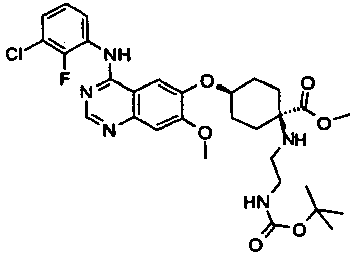 Massenspektrum (ESI⁺): m/z = 618, 620 [M+H]⁺

### Beispiel III

### trans-1-tert.-Butoxycarbonylamino-4-[4-(3-chlor-2-fluor-phenylamino)-7-methoxy-chinazolin-6-yloxy]-cyclohexancarbonsäure-methylester

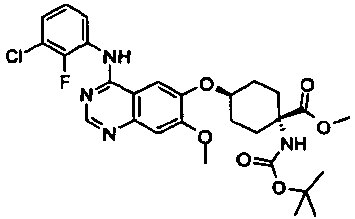

Ein Gemisch aus 3.30 g 4-(3-Chlor-2-fluor-phenylamino)-7-methoxy-chinazolin-6-ol in 23 ml N,N-Dimethylformamid wird auf 50 °C erwärmt. Dann werden 2.30 g Kaliumcarbonat und 4.40 g cis-1-tert.-Butoxycarbonylamino-4-methansulfonyloxy-cyclohexancarbonsäure-methylester zugesetzt. Das Reaktionsgemisch wird auf 80 °C erhitzt und über Nacht bei dieser Temperatur gerührt. Nun werden nochmals 1.00 g cis-1-tert.-Butoxycarbonylamino-4-methansulfonyloxy-cyclohexancarbonsäure-methylester und 0.90 g Kaliumcarbonat zugesetzt. Nach weiteren vier Stunden bei 80 °C wird das Reaktionsgemisch auf Raumtemperatur abgekühlt, mit Essigester verdünnt und mehrmals mit Wasser gewaschen. Die organische Phase wird mit gesättigter Natriumchlorid-Lösung gewaschen, über Magnesiumsulfat getrocknet und eingeengt. Der Kolbenrückstand wird chromatographisch über eine Kieselgelsäule mit Methylenchlorid/Methanol/konz. Ammoniak (98/2/0.1 auf 8/2/0.1) gereinigt.
Ausbeute: 5.50 g (93 % der Theorie)
Massenspektrum (ESI⁺): m/z = 575, 577 [M+H]⁺

### Beispiel IV

### cis-1-tert.-Butoxycarbonylamino-4-methansulfonyloxy-cyclohexancarbonsäure methylester

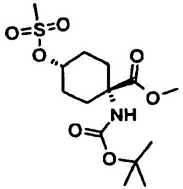

Zu 4.39 g cis-1-tert.-Butoxycarbonylamino-4-hydroxy-cyclohexancarbonsäure-methylester und 2.80 ml Triethylamin in 45 ml Methylenchlorid werden unter Eisbadkühlung langsam 1.40 ml Methansulfonsäurechlorid getropft, wobei die Temperatur unter 10 °C gehalten wird. Anschließend lässt man das Reaktionsgemisch auf Raumtemperatur kommen und rührt über Nacht. Nun werden 20 ml gesättigte Natriumhydrogencarbonat-Lösung zugegeben, die Phasen getrennt und die wässrige Phase wird mit Methylenchlorid extrahiert. Die vereinigten organischen Phasen werden mit gesättigter Natriumchlorid-Lösung gewaschen, über Magnesiumsulfat getrocknet und eingeengt, wobei ein zähes Öl zurückbleibt. Das Rohprodukt wird ohne weitere Reinigung weiter umgesetzt.
Ausbeute: 5.44 g (96 % der Theorie)
R_{f}-Wert: 0.50 (Kieselgel, Methylenchlorid/Methanol = 95:5)

Massenspektrum (ESI⁺): m/z = 352 [M+H]⁺

### Beispiel V

### cis-1-tert.-Butoxycarbonylamino-4-hydroxy-cyclohexancarbonsäure-methylester

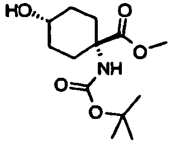

4.50 g 1-tert.-Butoxycarbonylamino-4-oxo-cyclohexancarbonsäure-methylester in 45 ml Tetrahydrofuran werden unter Argonatmosphäre mit 6 ml Wasser und 630 mg Natriumborhydrid versetzt. Das Reaktionsgemisch wird zwei Stunden bei Raumtemperatur gerührt, mit Diethylether verdünnt, mit 1 N Salzsäure versetzt und gründlich verrührt. Die organische Phase wird abgetrennt, mit 10%iger Kaliumcarbonat-Lösung und gesättigter Natriumchlorid-Lösung gewaschen, über Magnesiumsulfat getrocknet und eingeengt. Es bleibt ein farbloses Öl zurück, welches über Nacht langsam kristallisiert.
Ausbeute: 4.39 g (97 % der Theorie)
Massenspektrum (ESI⁺): m/z = 274 [M+H]⁺

### Beispiel VI

### 1-tert.-Butoxycarbonylamino-4-oxo-cyclohexancarbonsäure-methylester

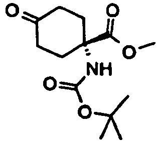

Zu 4.65 g 1-tert.-Butoxycarbonylamino-4-oxo-cyclohexancarbonsäure in 45 ml N,N-Dimethylformamid werden 3.90 g Kaliumcarbonat und 1.30 ml Methyliodid gegeben und das Reaktionsgemisch wird drei Stunden bei Raumtemperatur gerührt. Anschließend wird das Lösungsmittel am Rotationsverdampfer abdestilliert und der Rückstand zwischen 10%iger Kaliumcarbonat-Lösung und Diethylether verteilt. Die wässrige Phase wird abgetrennt und mit Diethylether extrahiert und die vereinigten organischen Phasen werden mit gesättigter Natriumchlorid-Lösung gewaschen, über Magnesiumsulfat getrocknet und eingeengt. Es bleibt ein farbloses Öl zurück, welches langsam kristallisiert.
Massenspektrum (ESI⁺): m/z = 272 [M+H]⁺

### Beispiel VII

### cis-1-Amino-4-[4-(3-chlor-2-fluor-phenylamino)-7-methoxy-chinazolin-6-yloxy]-cyclohexancarbonsäure-methylester

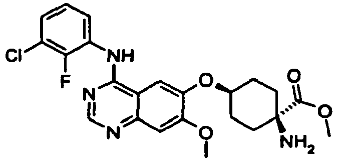

1.95 g cis/trans-1-Amino-4-[4-(3-chlor-2-fluor-phenylamino)-7-methoxy-chinazolin-6-yloxy]-cyclohexancarbonsäure werden in wenig Methanol gelöst und unter Eisbad-Kühlung zu einer Lösung aus 700 µl Thionylchlorid in 20 ml Methanol getropft. Das Reaktionsgemisch wird unter Rühren über Nacht auf Raumtemperatur erwärmt und anschließend noch 2 h unter Rückfluss erhitzt. Da noch keine Umsetzung zu erkennen ist, wird das Reaktionsgemisch abgekühlt, unter Eisbad-Kühlung mit weiteren 700 µl Thionylchlorid versetzt und erneut 8 h unter Rückfluss erhitzt. Da nach Abkühlung auf Raumtemperatur die Umsetzung immer noch nicht vollständig ist, werden nochmals 700 µl Thionylchlorid unter Eisbad-Kühlung zugesetzt. Nach weiteren 4 h unter Rückfluss ist die Umsetzung vollständig und das Lösungsmittel wird am Rotationsverdampfer abdestilliert. Der Kolbenrückstand wird zwischen Methylenchlorid und 10%iger Kaliumcarbonat-Lösung verteilt. Die wässrige Phase wird abgetrennt und mit Methylenchlorid extrahiert. Die vereinigten organischen Phasen werden über Magnesiumsulfat getrocknet und eingeengt. Der Kolbenrückstand wird über eine Reversed Phase-Säule mit einem Acetonitril/Wasser/Ammoniak-Gemisch als Laufmittel chromatographiert, wobei cis- und trans-Verbindung getrennt werden können.
Ausbeute: 330 mg (16 % der Theorie)
Massenspektrum (ESI⁺): m/z = 475, 477 [M+H]⁺

### Beispiel VIII

### cis/trans-1-Amino-4-[4-(3-chlor-2-fluor-phenylamino)-7-methoxy-chinazolin-6-yloxy] cyclohexancarbonsäure

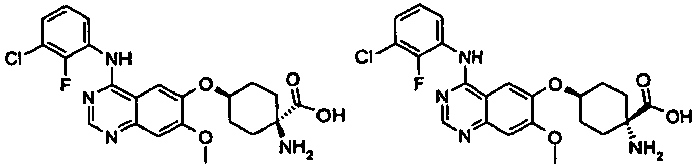

Ein Gemisch aus 3.00 g syn/anti-8-[4-(3-Chlor-2-fluor-phenylamino)-7-methoxy-chinazolin-6-yloxy]-1,3-diaza-spiro[4.5]decan-2,4-dion und 30 ml 2 N Natronlauge wird unter Rühren ca. 25 h auf 135 °C erhitzt. Nach Abkühlung auf Raumtemperatur wird das Reaktionsgemisch mit Salzsäure neutralisiert. Anschließend wird das Wasser am Rotationsverdampfer im Vakuum abdestilliert, wobei sich ein Niederschlag bildet, welcher abgesaugt und getrocknet wird. Das so erhaltene Rohprodukt wird ohne weitere Reinigung weiter umgesetzt.
Ausbeute: 1.95 g (69 % der Theorie)
Massenspektrum (ESI⁺): m/z = 461, 463 [M+H]⁺

### Beispiel IX

### syn/anti-8-[4-(3-Chlor-2-fluor-phenylamino)-7-methoxy-chinazolin-6-yloxy]-1,3-diazaspiro[4.5]decan-2,4-dion

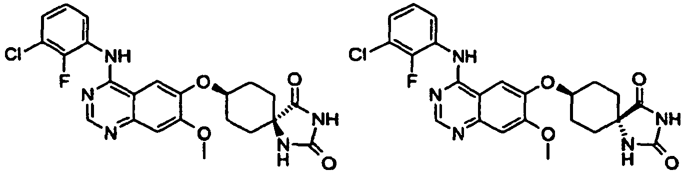

Eine Suspension von 3.00 g 4-[4-(3-Chlor-2-fluor-phenylamino)-7-methoxy-chinazolin-6-yloxy]-cyclohexanon in 30 ml 60%igem wässrigen Ethanol wird mit 2.10 g Ammoniumcarbonat und 470 mg Kaliumcyanid versetzt und das Reaktionsgemisch wird 2 h unter Rückfluss erhitzt. Nach Abkühlung auf Raumtemperatur werden nochmals 0.50 g Ammoniumcarbonat und 100 mg Kaliumcyanid zugegeben und das Reaktionsgemisch wird weitere 2 h unter Rückfluss erhitzt. Anschließend lässt man über Nacht auf Raumtemperatur abkühlen, wobei sich ein heller Niederschlag bildet. Dieser wird abgesaugt, mit Wasser nachgewaschen und getrocknet.
Ausbeute: 3.05 g (87 % der Theorie)
Massenspektrum (ESI⁺): m/z = 486, 488 [M+H]⁺

### Beispiel X

### 4-[4-(3-Chlor-2-fluor-phenylamino)-7-methoxy-chinazolin-6-yloxy]-cyclohexanon

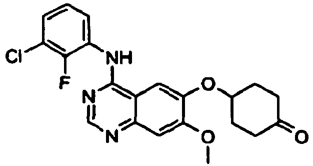

Zu 6.50 g 6-(1,4-Dioxa-spiro[4.5]dec-8-yloxy)-7-methoxy-3H-chinazolin-4-on in 65 ml Acetonitril werden unter Argonatmosphäre 3.25 ml Phosphoroxychlorid getropft. Anschließend wird das Reaktionsgemisch auf 40 °C erwärmt, tropfenweise mit 5.00 ml Triethylamin versetzt und 2 h unter Rückfluss erhitzt. Nach Abkühlung auf Raumtemperatur werden 1.40 ml Triethylamin und 2.60 ml 3-Chlor-2-fluor-anilin, gelöst in 5 ml Acetonitril, zugegeben und das Reaktionsgemisch wird über Nacht bei 40 °C gerührt. Dann werden nochmals 0.70 ml 3-Chlor-2-fluor-anilin, gelöst in 2 ml Acetonitril zugetropft und das Reaktionsgemisch wird weitere 10 h gerührt. Nach Abkühlung auf Raumtemperatur wird der entstandene Niederschlag abgesaugt, in 1 N Salzsäure aufgenommen, mit 6 N isopropanolischer Salzsäure versetzt und bei Raumtemperatur gerührt, bis die Ketalspaltung vollständig ist. Der entstandene Niederschlag wird abgesaugt und mit Methylenchlorid und 1 N Natronlauge versetzt. Die wässrige Phase wird abgetrennt und mit Methylenchlorid extrahiert, die vereinigten Extrakte werden eingeengt und der Kolbenrückstand wird mit Diisopropylether zur Kristallisation gebracht.
Ausbeute: 5.90 g (73 % der Theorie)
Massenspektrum (ESI⁺): m/z = 416, 418 [M+H]⁺

Analog Beispiel X werden folgende Verbindungen erhalten:
1) 4-[(2-Fluor-5-methyl-phenyl)amino]-6-(4-oxo-cyclohexyloxy)-7-methoxy-chinazolin 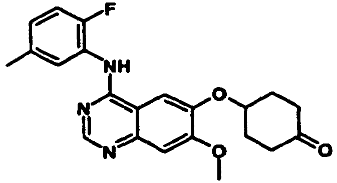 Massenspektrum (ESI⁺): m/z = 396 [M+H]⁺
(2)4-[(2,4-Difluor-3-methyl-phenyl)amino]-6-(4-oxo-cyclohexyloxy)-7-methoxy-chinazolin 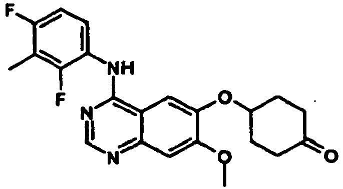 Massenspektrum (ESI⁺): m/z = 414 [M+H]⁺
(3) 4-[(2-Fluor-3-methyl-phenyl)amino]-6-(4-oxo-cyclohexyloxy)-7 -methoxy-chinazolin 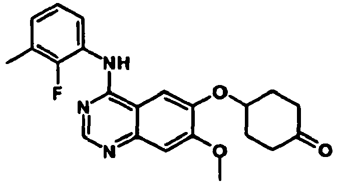 Massenspektrum (ESI⁺): m/z = 396 [M+H]⁺
(4) 4-[(3-Chlor-2-methyl-phenyl)amino]-6-(4-oxo-cyclohexyloxy)-7-methoxy-chinazolin 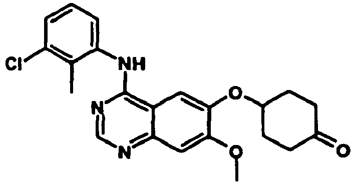 Massenspektrum (ESI⁺): m/z = 412, 414 [M+H]⁺
(5) 4-[(5-Chlor-2-fluor-phenyl)amino]-6-(4-oxo-cyclohexyloxy)-7-methoxy-chinazolin 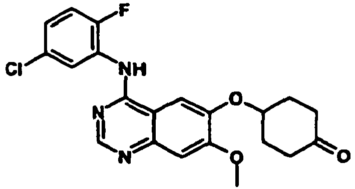 Massenspektrum (ESI⁺): m/z = 416, 418 [M+H]⁺
(6) 4-[(4-Fluor-3-methyl-phenyl)amino]-6-(4-oxo-cyclohexyloxy)-7-methoxy-chinazolin 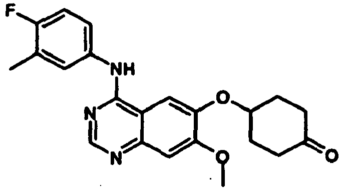 Massenspektrum (ESI⁺): m/z = 396 [M+H]⁺
(7) 4-[(3-Fluor-5-methyl-phenyl)amino]-6-(4-oxo-cyclohexyloxy)-7-methoxy-chinazolin 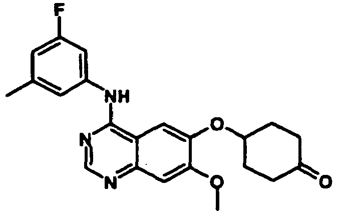 Massenspektrum (ESI⁺): m/z = 396 [M+H]⁺
(8) (R)-4-[(1-Phenylethyl)amino]-6-(4-oxo-cyclohexyloxy)-7-methoxy-chinazolin 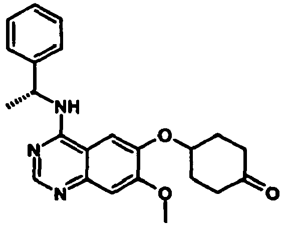 Massenspektrum (ESI⁺): m/z = 392 [M+H]⁺

### Beispiel XI

### 6-(1,4-Dioxa-spiro[4.5]dec-8-yloxy)-7-methoxy-3H-chinazolin-4-on

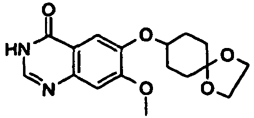

27.20 g 3-Benzyl-6-(1,4-dioxa-spiro[4.5]dec-8-yloxy)-7-methoxy-3H-chinazolin-4-on werden in 270 ml Eisessig gelöst, mit 2.70 g Palladium auf Aktivkohle (10 %ig) versetzt und bei 60 °C hydriert, bis die Wasserstoffaufnahme beendet ist. Anschließend wird der Eisessig am Rotationsverdampfer abdestilliert und mit Toluol nachgedampft. Der Kolbenrückstand wird mit Wasser versetzt und mit gesättigter Natriumhydrogencarbonat-Lösung alkalisch gestellt. Der entstandene Niederschlag wird abgesaugt und getrocknet.
Ausbeute: 20.30 g (95 % der Theorie)
Massenspektrum (ESI⁺): m/z = 333 [M+H]⁺

### Beispiel XII

### 3-Benzyl-6-(1,4-dioxa-spiro[4.5]dec-8-yloxy)-7-methoxy-3H-chinazolin-4-on

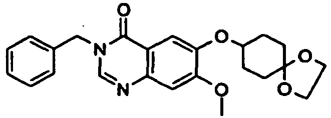

20.00 g 3-Benzyl-6-hydroxy-7-methoxy-3H-chinazolin-4-on in 150 ml N,N-Dimethylformamid werden auf 50 °C erwärmt, dann werden 16.00 g Kaliumcarbonat und 20.00 g Methansulfonsäure-1,4-dioxa-spiro[4.5]dec-8-ylester zugegeben und das Reaktionsgemisch über Nacht bei 80 °C gerührt. Dann werden nochmals 6.00 g Kaliumcarbonat und 8.00 g Methansuifonsäure-1,4-dioxa-spiro[4.5]dec-8-ylester zugesetzt und es wird weitere 4 h bei 80 °C gerührt. Innerhalb der nächsten 24 h werden noch insgesamt weitere 6.00 g Kaliumcarbonat und 10.00 g Methansulfonsäure-1,4-dioxa-spiro[4.5]dec-8-ylester portionsweise zugegeben, bis die Umsetzung vollständig ist. Nach Abkühlung auf Raumtemperatur werden sehr langsam unter Rühren insgesamt 450 ml Wasser zugetropft, wobei sich ein Niederschlag bildet, welcher abgesaugt, mit Wasser gewaschen und getrocknet wird.
Ausbeute: 27.20 g (91 % der Theorie)
Massenspektrum (ESI⁺): m/z = 423 [M+H]⁺

### Herstellung der Endverbindungen

### Beispiel 1

### anti-9-[4-(3-Chlor-2-fluor-phenylamino)-7-methoxy-chinazolin-6-yloxy]-1,4-diaza-spiro[5.5]undecan-5-on

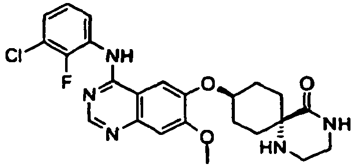

Zu 1.30 g trans-1-(2-Amino-ethylamino)-4-[4-(3-chlor-2-fluor-phenylamino)-7-methoxy-chinazolin-6-yloxy]-cyclohexancarbonsäure-methylester in 14 ml Methanol werden 1.30 ml 4 N Natronlauge gegeben und das Reaktionsgemisch wird drei Stunden bei Raumtemperatur gerührt. Anschließend wird das Lösungsmittel am Rotationsverdampfer im Vakuum abdestilliert. Der Kolbenrückstand wird chromatographisch über eine Kieselgelsäule mit Methylenchlorid/Methanollkonz. Ammoniak (98/2/0.1 auf 8/2/0.1) als Laufmittel gereinigt. Die Produktfraktionen werden eingeengt und mit Diisopropylether verrührt. Der feste Rückstand wird abgesaugt und getrocknet.
Ausbeute: 700 mg (57 % der Theorie)
R_{f}-Wert: 0.30 (Kieselgel, Methylenchlorid/Methanol/konz. Ammoniak = 90:10:1)
Massenspektrum (ESI⁺): m/z = 486, 488 [M+H]⁺

Analog Beispiel 1 werden folgende Verbindungen erhalten:
(1) anti-9-[4-(3-Chlor-2-fluor-phenylamino)-7-methoxy-chinazolin-6-yloxy]-4-methyl-1,4-diaza-spiro[5.5]undecan-5-on 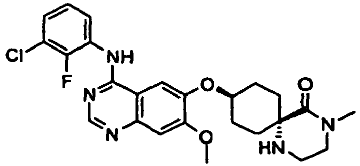 Massenspektrum (ESI⁺): m/z = 500, 502 [M+H]⁺
(2) syn-9-[4-(3-Chlor-2-fluor-phenylamino)-7-methoxy-chinazolin-6-yloxy]-1.4-diaza-spiro[5.5]undecan-5-on 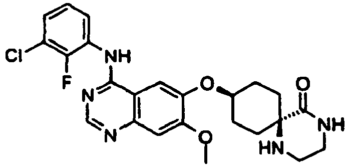 Massenspektrum (ESI⁺): m/z = 486, 488 [M+H]⁺

### Beispiel 2

### anti-9-[4-(3-Chlor-2-fluor-phenylamino)-7-methoxy-chinazolin-6-yloxy]-1-methyl-1.4-diaza-spiro[5.5]undecan-5-on

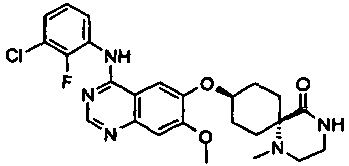

Zu 400 mg anti-9-[4-(3-Chlor-2-fluor-phenylamino)-7-methoxy-chinazolin-6-yloxy]-1,4-diaza-spiro[5.5]undecan-5-on in 12 ml Tetrahydrofuran werden 125 µl 37%ige wässrige Formaldehyd-Lösung, gefolgt von 50 µl Eisessig und 280 mg Natriumtriacetoxyborhydrid gegeben. Das Reaktionsgemisch wird über Nacht bei Raumtemperatur gerührt, mit Essigester verdünnt, mit 1 N Natronlauge versetzt und verrührt. Die organische Phase wird abgetrennt, mit Wasser und gesättigter Natriumchlorid-Lösung gewaschen, über Magnesiumsulfat getrocknet und eingeengt. Der Kolbenrückstand wird chromatographisch über eine Kieselgelsäule mit Methylenchlorid/Methanol/konz. Ammoniak (99/1/0.2 auf 8/2/0.1) als Laufmittel gereinigt. Das Rohprodukt wird mit Methanol verrührt, abgesaugt und getrocknet.
Ausbeute: 230 mg (56 % der Theorie)
Massenspektrum (ESI⁺): m/z = 500, 502 [M+H]⁺

Analog Beispiel 2 wird folgende Verbindung erhalten:
(1) anti-9-[4-(3-Chlor-2-fluor-phenylamino)-7-methoxy-chinazolin-6-yloxy]-1,4-dimethyl-1,4-diaza-spiro[5.5]undecan-5-on 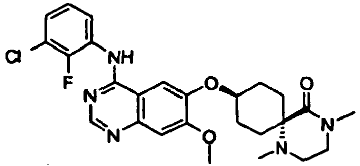 Massenspektrum (ESI⁺): m/z = 514, 516 [M+H]⁺

### Beispiel 3

### syn/anti-8-[4-(3-Chlor-2-fluor-phenylamino)-7-methoxy-chinazolin-6-yloxy]-1.3-diaza-spiro[4.5]decan-2,4-dion

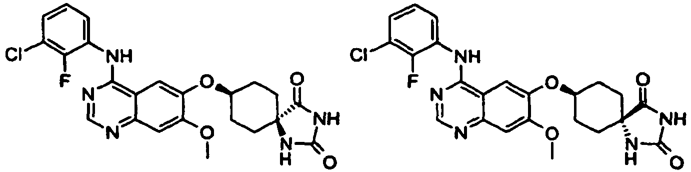

Eine Suspension von 3.00 g 4-[4-(3-Chlor-2-fluor-phenylamino)-7-methoxy-chinazolin-6-yloxy]-cyclohexanon in 30 ml 60%igem wässrigen Ethanol wird mit 2.10 g Ammoniumcarbonat und 470 mg Kaliumcyanid versetzt und das Reaktionsgemisch wird 2 h unter Rückfluss erhitzt. Nach Abkühlung auf Raumtemperatur werden nochmals 0.50 g Ammoniumcarbonat und 100 mg Kaliumcyanid zugegeben und das Reaktionsgemisch wird weitere 2 h unter Rückfluss erhitzt. Anschließend lässt man über Nacht auf Raumtemperatur abkühlen, wobei sich ein heller Niederschlag bildet. Dieser wird abgesaugt, mit Wasser nachgewaschen und getrocknet.
Ausbeute: 3.05 g (87 % der Theorie)
Massenspektrum (ESI⁺): m/z = 486, 488 [M+H]⁺

### Biologischer Test

Die biologischen Eigenschaften der neuen Verbindungen werden beispielsweise wie folgt geprüft:

Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Eine murine hämatopoetische Zelllinie wird derart genetisch verändert, dass sie funktionellen humanen EGF-R exprimiert. Die Proliferation dieser Zelllinie kann daher durch EGF stimuliert werden.

### Der Test wird wie folgt durchgeführt:

Die Zellen werden in RPMI/1640 Medium kultiviert. Die Proliferation wird mit 20 ng/ml humanem EGF (Promega) stimuliert. Zur Untersuchung der inhibitorischen Aktivität der erfindungsgemäßen Verbindungen werden diese Verbindungen in 100% Dimethylsulfoxid (DMSO) gelöst und in verschiedenen Verdünnungen den Kulturen zugefügt, wobei die maximale DMSO Konzentration 1% beträgt. Die Kulturen werden für 48 Stunden bei 37°C inkubiert.

Zur Bestimmung der inhibitorischen Aktivität der erfindungsgemäßen Verbindungen wird die relative Zellzahl mit dem Cell Titer 96TM AQueous Non-Radioactive Cell Proliferation Assay (Promega) in O.D. Einheiten gemessen. Die relative Zellzahl wird in Prozent der Kontrolle berechnet und die Wirkstoffkonzentration, die die Proliferation der Zellen zu 50% hemmt (IC50), abgeleitet.

Die erfindungsgemäßen Verbindungen der allgemeinen Formel **(I)** zeigen beispielsweise IC50-Werte von < 10 micromolar, vorzugsweise von < 1 micromolar.

| Verbindung (Beispiel Nr.) | Hemmung der EGFR-abhängigen Proliferation IC₅₀ [nM] |
|---|---|
| 1 | 4 |
| 1(1) | 2 |
| 1(2) | 2 |
| 2 | 1 |
| 2(1) | 2 |

### Indikationsgebiete

Wie gefunden wurde, zeichnen sich die Verbindungen der Formel (I) durch vielfältige Anwendungsmöglichkeiten auf therapeutischem Gebiet aus. Hervorzuheben sind solche Anwendungsmöglichkeiten, für welche die erfindungsgemäßen Verbindungen der Formel (I) aufgrund ihrer pharmazeutischen Wirksamkeit als Tyrosinkinase-Hemmer bevorzugt zur Anwendung gelangen können.

Die erfindungsgemäßen Verbindungen der allgemeinen Formel (I) hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuro-epithelialen Ursprungs, Metastasierung sowie die abnorme Proliferation vaskulärer Endothelzellen (Neoangiogenese). Darüber hinaus sind EGFR Inhibitoren nützlich zur Behandlung von viralen Infektionen, bei denen das Virus für den Eintritt bzw. Befall der Zelle oder zu seiner Vermehrung oder für die Reaktion des Wirtes auf das Virus den Signaltransduktionsweg des EGFR verwendet.

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen. Weiterhin sind die Verbindungen nützlich zur Behandlung von viralen oder bakteriellen Exazerbationen, sowie zur Behandlungen von viralen oder bakteriellen Infektionen der Atemwege oder Lunge, wenn bei Eintritt, Vermehrung oder der Gewebereaktion des Wirtes die Tyrosinkinase aktiviert wird.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezemierende Adenome und Proteinverlustsyndrome.

Außerdem können die Verbindungen der allgemeinen Formel (I) und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH). inflammatorischer Prozesse, Erkrankungen des Immunsystems, Hyperproliferation hämatopoetischer Zellen, der Behandlung von Nasenpolypen, etc..

Die Verbindungen der Formel (I) können allein oder in Kombination mit anderen Wirkstoffen der Formel (I) zur Anwendung gelangen. Gegebenenfalls können die Verbindungen der Formel (I) auch in Kombination mit **W** eingesetzt werden, worin **W** einen pharmakologisch, aktiven Wirkstoff darstellt und beispielsweise ausgewählt ist, aus der Gruppe bestehend aus Betamimetika, Anticholinergika, Corticosteroiden, PDE4-Inhibitoren, LTD4-Rezeptor (CysLT1, CysLT2, CysLT3) Antagonisten, EGFR-Hemmem, Dopamin-Agonisten, H1-Antihistaminika, PAF-Antagonisten, SYK-Inhibitoren, PDE3 Inhibitoren, Lipoxin A4 Derivate, FPRL1 Modulatoren, LT84-Rezeptor (BLT1, BLT2) Antagonisten, Histamin H1 Rezeptor Antagonisten, Histamin H4 Rezeptor Antagonisten, PI3 Kinase Inhibitoren, Inhibitoren von Nicht-Rezeptor Tyrosine Kinasen wie zum Beispiel LYN, LCK, SYK, ZAP-70, FYN, BTK oder ITK, Inhibitoren von MAP Kinasen wie zum Beispiel p38, ERK1, ERK2, JNK1, JNK2, JNK3 oder SAP, Inhibitoren des NF-kappaB Signalwegs wie zum Beispiel IKK Kinase Inhibitoren, iNOS Inhibitoren, MRP4 Inhibitoren, Leukotriene Biosynthese Inhibitoren wie zum Beispiel 5-Lipoxygenase (5-LO) Inhibitoren, cPLA2 Inhibitoren, Leukotriene A4 Hydrolase Inhibitoren oder FLAP Inhibitoren, Nicht-steroidale antientzündliche Agenzien (NSAIDs), CRTH2 Antagonisten, DP1-Rezeptor Modulatoren, Thromboxane Rezeptor Antagonisten, Chemokine Rezeptor Antagonisten von CCR1, CCR2, CCR2A, CCR2B, CCR4, CCR5, CCR6" CCR7, CCRB, CCR9, CCR10, CCR11, CXCR1, CXCR2, CXCR3, CXCR4, CXCR5, CXCR6, CX3CR1, Neurokinin (NK1, NK2) Antagonisten, Sphingosine 1-Phosphat Rezeptor Modulatoren, Modulatoren von Adenosine Rezeptoren, Modulatoren von purinergen Rezeptoren wie zum Beispiel P2X7, Histone Deacetylase (HDAC) Aktivatoren, Bradykinin (BK1, BK2) Antagonisten, TACE Inhibitoren, Mucoregulatoren, PPAR gamma Agonisten, Rho Kinase Inhibitoren, interleukin 1-beta converting enzyme (ICE) Inhibitoren, Toll-Like Rezeptor (TLR) Modulatoren, HMG-CoA Reductase Inhibitoren, VLA-4 Antagonisten, ICAM-1 Inhibitoren, SHIP Agonisten, TNFalpha Antagonisten, GABAa Rezeptor Antagonisten, Immunotherapeutika, Substanzen gegen Schwellungen der Atemwege und Substanzen gegen Husten.

Weiterhin können zwei- oder dreifach Kombinationen von **W** mit den Verbindungen der Formel (I) kombiniert werden. Beispielhaft genannte Kombinationen von **W** mit den Verbindungen der Formel 1 wären:
- **W** stellt ein Betamimetikum dar, kombiniert mit einem Anticholinergikum, Corticosteroid, PDE4-Inhibitor, EGFR-Hemmer, p38 MAP Kinase Hemmer oder LTD4-Rezeptor Antagonisten,
- **W** stellt ein Anticholinergikum dar, kombiniert mit einem Betamimetikum, Corticosteroid, PDE4-Inhibitor, EGFR-Hemmen, p38 MAP Kinase Hemmer oder LTD4-Rezeptor Antagonisten,
- **W** stellt ein Corticosteroid dar, kombiniert mit einem PDE4-Inhibitor, EGFR-Hemmer oder LTD4-Rezeptor Antagonist
- **W** stellt ein PDE4-Inhibitor dar, kombiniert mit einem EGFR-Hemmer, p38 MAP Kinase Hemmer oder LTD4- Rezeptor Antagonist oder p38 MAP Kinase Hemmer
- **W** stellt ein EGFR-Hemmer dar, kombiniert mit einem Anticholinergikum.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenatine, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, und 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1 H-chinolin-2-on, 4-Hydroxy-7-[2-{[2-{[3-(2-phenylethoxy)propyl]sulfonyl}ethyl]-amino}ethyl]-2(3H)-benzothiazolon, 1-(2-Fluor-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazo1-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chlor-5-trifluormethylphenyl)-2-tert.-butylamino)ethanol, 6-Hydroxy-8-(1-hydroxy-2-[2-(4-methoxy-phenyl)-1,1-dimethyl-ethylamino]-ethyl}-4H-benzo[1,4]oxazin-3-on 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1.4]oxazin-3-on. 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1 dimethyl-ethylamino]-ethyl}-4H-benzo[1.4]oxazin-3-on, 8-{2-(2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluorphenyl)-1,1-dimethyl-ethylamino]-1-hydroxy-ethyl}-6-hydroxy-4H-benzo[1,4]oxazin-3-on, 1-(4-Ethoxy-carbonylamino-3-cyano-5-fluorophenyl)-2-(tert-butylamino)ethanol, 2-Hydroxy-5-(1-hydroxy-2-{2-[4-(2-hydroxy-2-phenyl-ethylamino)-phenyl]-ethylamino}-ethyl)-benzaldehyd, N-[2-Hydroxy-5-(1-hydroxy-2-{2-[4-(2-hydroxy-2-phenyl-ethylamino)-phenyl]-ethylamino}-ethyl)-phenyl]-formamid, 8-Hydroxy-5-(1-hydroxy-2-{2-[4-(6-methoxy-biphenyl-3-ylamino)-phenyl]-ethylamino}-ethyl)-1 H-chinolin-2-on, 8-Hydroxy-5-[1-hydroxy-2-(6-phenethylamino-hexylamino)-ethyl]-1 H-chinolin-2-one, 5-[2-(2-{4-[4-(2-Amino-2-methyl-propoxy)-phenylamino]-phenyl}-ethylamino)-1-hydroxy-ethyl]-8-hydroxy-1H-chinolin-2-on, [3-(4-{6-[2-Hydroxy-2-(4-hydroxy-3-hydroxymethyl-phenyl)-ethylamino]-hexyloxy}-butyl)-5-methyl-phenyl]-hamstoff, 4-(2-{6-[2-(2,6-Dichloro-benzyloxy)-ethoxy]-hexylamino}-hydroxy-ethyl)-2-hydroxymethyl-phenol, 3-(4-{6-[2-Hydroxy-2-(4-hydroxy-3-hydroxymethyl-phenyl)-ethylamino]-hexyloxy}-butyl)-benzenesulfonamid, 3-(3-{7-[2-Hydroxy-2-(4-hydroxy-3-hydroxymethyl-phenyl)-ethylamino]-heptyloxy}-propyl)-benzenesulfonamid, 4-(2-{6-[4-(3-Cyclopentanesulfonyl-phenyl)-butoxyl-hexylamino}-1-hydroxy-ethyl)-2-hydroxymethyl-phenol, N-Adamantan-2-yl-2-(3-{2-[2-hydroxy-2-(4-hydroxy-3-hydroxymethyl-phenyl)-ethylamino]-propyl}-phenyl)-acetamid, (R,S)-4-(2-{[6-(2,2-Difluoro-4-phenylbutoxy)hexyl]amino}-1-hydroxy-ethyl)-2-(hydroxymethyl)phenol, (R,S)-4-(2-{[6-(2,2-Difluoro-2-phenylethoxy)hexyl]amino}-1-hydroxy-ethyl)-2-(hydroxymethyl)phenol, (R,S)-4-(2-{[4,4-Difluoro-6-(4-phenylbutoxy)hexyl]amino}-1-hydroxy-ethyl)-2- (hydroxymethyl)phenol, (R,S)-4-(2-{[6-(4,4-Difluoro-4-phenylbutoxy)hexyl]amino}-1-hydroxy-ethyl)-2-(hydroxymethyl)phenol, (R,S)-5-(2-{[6-(2,2-Difluoro-2-phenylethoxy)hexyl]amino}-1-hydroxyethyl)-8- hydroxychinolin-2(1 H)-on, (R,S)-[2-({6-[2,2-Difluoro-2-(3-methylphenyl)ethoxy]hexyl}amino)-1-hydroxyethyl]-2-(hydroxymethyl)phenol, 4-(1R)-2-{[6-(2,2-Difluoro-2-phenylethoxy)hexyl]amino}-1-hydroxyethyl)-2-(hydroxymethyl)phenol, (R,S)-2-(Hydroxymethyl)-4-(1-hydroxy-2-{[4,4,5I5-tetrafluoro-6-(3-phenylpropoxy)hexyl]amino}ethyl)phenol, (R,S)-[5-(2-{[6-(2,2-Difluoro-2-phenylethoxy)hexyl]amino}-1-hydroxy-ethyl)-2- hydroxyphenyl]formamid, (R,S)-4-[2-({6-[2-(3-Bromophenyl)-2,2-difluoroethoxy]hexyl}amino)-1-hydroxyethyl]- 2-(hydroxymethyl)phenol, (R, S)-N-[3-(1,1 -Difluoro-2-{[6-({2-hydroxy-2-[4-hydroxy-3-(hydroxymethyl)phenyl]-ethyl}amino)hexyl]oxy}ethyl)phenyl]harnstoff, 3-[3-(1,1-difluoro-2-{[6-({2-hydroxy-2-[4-hydroxy-3-(hydroxymethyl) phenyl]ethyl}amino)hexyl]oxy)ethyl)phenyl]imidazolidine-2,4-dion, (R,S)-4-[2-({6-[2,2-difluoro-2-(3-methoxyphenyl)ethoxy]hexyl}amino}-1- hydroxyethyl]-2-(hydroxymethyl)phenol, 5-((1R)-2-{[6-(2,2-difluoro-2-phenylethoxy)hexyl]amino}-1-hydroxyethyl)-8- hydroxychinolin-2(1 H)-on, 4-((1 R)-2-{[4,4-Difluoro-6-(4-phenylbutoxy)hexyl]amino}-1-hydroxy-ethyl)-2-(hydroxymethyl)phenol, (R,S)-4-(2-{[6-(3,3-Difluoro-3-phenylpropoxy)hexyl]amino}-1-hydroxy ethyl)-2-(hydroxymethyl)phenol, (R,S)-(2-{[6-(2,2-Difluoro-2-phenylethoxy)-4,4-difluorohexyl]amino}-1-hydroxyethyl)-2-(hydroxymethyl)phenol und (R,S)-4-(2-{[6-(2,2-difluoro-3-phenylpropoxy)hexyl]amino}-1-hydroxy-ethyl)-2-(hydroxymethyl)phenol,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus: Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3R)-1-Phenethyl-3-(9H-xanthen-9-carbonyloxy)-1-azoniabicyclo[2.2.2]octan-Salze. In den vorstehend genannten Salzen stellen die Kationen die pharmakologisch aktiven Bestandteile dar. Als Anionen X-können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Weiterhin genannte Verbindungen sind: 2,2-Diphenylpropionsäuretropenolester-Methobromid, 2,2-Diphenylpropionsäurescopinester-Methobromid , 2-Fluor-2,2-Diphenylessigsäurescopinester-Methobromid, 2-Fluor-2.2-Diphenylessigsäuretropenolester Methobromid, 3,3',4,4'-Tetrafluorbenzilsäuretropenolester-Methobromid , 3,3',4,4'-Tetrafluorbenzilsäurescopinester-Methobromid, 4,4'-Difluorbenzilsäuretropenolester-Methobromid , 4,4'-Difluorbenzilsäurescopinester-Methobromid , 3,3'-Difluorbenzilsäuretropenolester-Methobromid , 3,3'-Difluorbenzilsäurescopinester-Methobromid, 9-Hydroxy-fluoren-9-carbonsäuretropenolester-Methobromid , 9-Fluor-fluoren-9-carbonsäuretropenolester-Methobromid , 9-Hydroxy-fluoren-9-carbonsäurescopinester-Methobromid , 9-Fluor-fluoren-9-carbonsäurescopinester-Methobromid , 9-Methyl-fluoren-9-carbonsäuretropenolester-Methobromid , 9-Methyl-fluoren-9-carbonsäurescopinester-Methobromid , Benzilsäurecyclopropyltropinester-Methobromid , 2,2-Diphenylpropionsäurecyclopropyltropinester-Methobromid, 9-Hydroxy-xanthen-9-carbonsäurecyclopropyltropinester-Methobromid , 9-Methyl-fluoren-9-carbonsäurecyclopropyttropinester-Methobrornid , 9-Methyl-xanthen-9-carbonsäurecyclopropyltropinester-Methobromid , 9-Hydroxy-fluoren-9-carbonsäurecyclopropyltropinester-Methobromid, 4,4'-Difluorbenzilsäuremethylestercyclopropyltropinester-Methobromid, 9-Hydroxy-xanthen-9-carbonsäuretropenolester-Methobromid , 9-Hydroxy-xanthen-9-carbonsäurescopinester-Methobromid , 9-Methyl-xanthen-9-carbonsäuretropenolester-Methobromid, 9-Methyl-xanthen-9-carbonsäurescopinester-Methobromid , 9-Ethyl-xanthen-9-carbonsäuretropenolester-Methobromid , 9-Difluormethyl-xanthen-9-carbonsäuretropenolester-Methobromid und 9-Hydroxymethyl-xanthen-9-carbonsäurescopinester-Methobromid
Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus: Bedomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, Tipredane und Pregna-1,4-diene-3,20-dione, 6-fluoro-11-hydroxy-16,17-[(1-methylethylidene)bis(oxy)]-21-[[4-[(nitrooxy)methyl]benzoyl]oxy]-, (6α,11β,16α)- (9CI) (NCX-1 024), 16,17-butylidenedioxy-6,9-difluoro-11-hydroxy-17-(methylthio)androst-4-en-3-one (RPR-106541), 6,9-Difluor-17-[(2-furanylcarbonyl)oxy]-11-hydroxy-16-methyl-3-oxo-androsta-1,4-dien-17-carbothionsäure (S)-fluoromethylester, 6,9-Difluor-11-hydroxy-16-methyl-3-oxo-17-propionyloxy-androsta-1,4-dien-17-carbothionsäure (S)-(2-oxo-tetrahydro-furan-3S-yl)ester, 6alpha,9alpha-difluoro-11 beta-hydroxy-16alpha-methyl-3-oxo-17alpha-(2,2,3,3-tetramethylcyclopropylcarbonyl)oxy-androsta-1,4-diene-17beta-carbonsäure cyanomethyl ester,
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast Pumafentrin , Lirimilast, Apremilast, Arofyllin, Atizoram, Oglemilast, Tetomilast, und 5-[(N-(2,5-dichloro-3-pyridinyl)-carboxamid)-8-methoxy-Chinolin (D-4418), 5-N-(3,5-dichloro-1-oxido-4-pyridinyl)-carboxamid]-8-methoxy-2-(trifluoromethyl)-Chinolin (D-4396 (Sch-351591)), N-(3,5-dichloropyrid-4-yl)-[1-(4-fluorobenzyl)-5-hydroxy-indol-3-yl]glyoxylsäureamid (AWD-12-281 (GW-842470)), 9-[(2-fluorophenyl)methyl]-N-methyl-2-(trifluoromethyl)-9H-Purin-6-amine (NCS-613), 4-[(2R)-2-[3-(cyclopentyloxy)-4-methoxyphenyl]-2-phenylethyl]-Pyridine (CDP-840), N-[(3R)-3,4,6,7-tetrahydro-9-methyl-4-oxo-1-phenylpyrrolo[3,2, 1-jk][1,4]benzodiazepin-3-yl]-4-Pyridinecarboxamide (PD-168787), 4-[6,7-diethoxy-2,3-bis(hydroxymethyl)-1-naphthalenyl]-1-(2-methoxyethyl)-2(1H)-Pyridinone (T-440), 2-[4-[6,7-diethoxy-2,3-bis(hydroxymethyl)-1-naphthalenyl]-2-pyridinyl]-4-(3-pyridinyl)-1 (2H)-Phthalazinone (T-2585), (3-(3-cyclopenyloxy-4-methoxybenzyl)-6-ethylamino-8-isopropyl-3H-purine (V-11294A), beta-[3-(cyclopentyloxy)-4-methoxyphenyl]-1,3-dihydro-1,3-dioxo-2H-Isoindole-2-propanamid (CDC-801), Imidazo[1,5-a]pyrido[3,2-e]pyrazin-6(5H)-one, 9-ethyl-2-methoxy-7-methyl-5-propyl- (D-22888)5-[3-(cyclopentyloxy)-4-methoxyphenyl]-3-[(3-methylphenyl)methyl]-, (3S,5S)-2-Piperidinon (HT-0712), 4-[1-[3,4-bis(difluoromethoxy)phenyl]-2-(3-methyl-1-oxido-4-pyridinyl)ethyl]-alpha,alpha-bis(trifluoromethyl)-Benzenemethanol (L-826141), N-(3,5-Dichloro-1-oxo-pyridin-4-yl)-4-difluormethoxy-3-cyclopropylmethoxybenzamid, (-)p-[(4aR*,10bS*)-9-Ethoxy-1,2,3,4,4a,1 0b-hexahydro-8-methoxy-2-methylbenzo[s][1,6]naphthyridin-6-yl]-N,N-diisopropylbenzamid, (R)-(+)-1-(4-Brombenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon and cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluormethoxyphenyl)cyclohexan-1-ol], (R)-(+)-Ethyl[4-(3-cyclopentyloxy-4-methoxyphenyl)pyrrolidin-2-yliden]acetate, (S)-(-)-Ethyl[4-(3-cyclopentyloxy-4-methoxyphenyl)pyrrolidin-2-yliden]acetate, 9-Cyclopentyl-5,6-dihydro-7-ethyl-3-(2-thienyl)-9H-pyrazolo[3,4-c]-1,2,4-triazolo[4,3-a]pyridine and 9-Cyclopentyl-5,6-dihydro-7-ethyl-3-(tert-butyl)-9H-pyrazolo[3,4-c]-1,2,4-triazolo[4,3-a]pyridin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als EGFR-Hemmer gelangen hierbei vorzugsweise Verbindungen zur Anwendung, die ausgewählt sind aus der Gruppe bestehend aus
Cetuximab, Trastuzumab, Panitumumab (=ABX-EGF), Mab ICR-62, Gefitinib, Canertinib, Erlotinib, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl}-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin 4-[(3-Chlor-4-fluorphenyl)amino]-6-({4-[N-(2-methoxy-ethyl)-N-methyl-amino]-1-oxo-2-buten-1-yi}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxyethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin , 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7 -cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluorphenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-pheny)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluor-phenyl)amino]-6-(piperidin-3-yloxy)-7 -methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-(S)-tetrahydrofuran-3-yloxy)-7 -hydroxychinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-(trans-4-[(morpholin-4-yl)sulfonylamino]-cyclohexan-1-yloxy)-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluorphenyl)amino]-6-(cis-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cisr-4-acetylamino-cyclohexan-1-yloxy)-7-methoxy-chinazolin, 4-[(3-Ethinyl-phenyl)amino]-6-[(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluorphenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)aminol-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-(1-[(S,S)-(2-oxa-5-aza-bicyclo[2.2.1]hept-5-yl)carbonyl]-piperidin-4-yloxy)-7-methoxy-chinazolin, 4-[(3-Chlor-4-fluorphenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluorphenyl)amino]-6-(trans-4-methylamino-cyclohexan-1-yloxy)-7-methoxy-chinazolin, 4-[(3-Chlor-4-fluor-phenyl)aminol-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxyl-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, 3-Cyano-4-[(3-chlor-4-fluorphenyl)amino]-6-{[4-(N,N-dimethylamino)-1-oxo-2-buten-1-yl]amino}-7-ethoxy-chinolin; [4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-quinazoline, 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-8-[(vinylcarbonyl)amino]-chinazolin, 4-[(3-Chlor-4-fluorphenyl)amino]-7-[4-((R)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, und 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Rezeptor Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als PAF-Antagonisten gelangen hierbei vorzugsweise Verbindungen zur Anwendung, die ausgewählt sind aus der Gruppe bestehend aus lexipafant und 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin, 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTB4-Rezeptor Antagonisten gelangen hierbei vorzugsweise Verbindungen zur Anwendung wie z.B. Amebulant (=[[4-[[3-[[4-[1-(4-hydroxyphenyl)-1-methylethyl]phenoxy]methyl]phenyl]methoxy]phenyl]iminomethyl]-Carbaminsäure-ethyl ester), gegebenenfalls in Form ihrer Racemate, Enantiomere, Diastereomere und gegebenenfalls in Form ihrer pharmakologisch verträglichen Säureadditionssalze, Solvate, Prodrugs oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Rezeptor Antagonisten gelangen hierbei vorzugsweise Verbindungen zur Anwendung, die ausgewählt sind aus der Gruppe bestehend aus Montelukast, Pranlukast, Zafirlukast, und (E)-8-[2-[4-[4-(4-Fluorophenyl)butoxy]phenyl]ethenyl]-2-(1H-tetrazol-5-yl)-4H-1-benzopyran-4-one (MEN-91507), 4-[6-Acetyl-3-[3-(4-acetyl-3-hydroxy-2-propylphenylthio)propoxy]-2-propylphenoxy]Buttersäure (MN-001), 1-(((R)-(3-(2-(6,7-Difluor-2-chinolinyl)ethenyl)phenyl)-3-(2-(2-hydroxy-2-propyl)phenyl)thio)methylcyclopropan-essigsäure,, 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure, [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als Histamin H1 Rezeptor Antagonisten gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxatat, Hydrosuccinat, Hydrobenzoat und Hydro-p-toluolsulfonat.

Als Histamin H4 Rezeptor Antagonisten gelangen hierbei vorzugsweise Verbindungen zur Anwendung wie z.B. (5-chloro-1H-indol-2-yl)(4-methyl-1-piperazinyl)-Methanone (JNJ-7777120), gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als Inhibitoren von Nicht-Rezeptor Tyrosine Kinasen wie zum Beispiel LYN, LCK, SYK, ZAP-70, FYN, BTK oder ITK gelangen hierbei vorzugsweise Verbindungen zur Anwendung, die ausgewählt sind aus der Gruppe bestehend aus 2-[(2-aminoethyl)amino]-4-[(3-bromophenyl)amino]-5-Pyrimidinecarboxamid; 2-[[7-(3,4-dimethoxyphenyl)imldazo[1,2-c]pyrimidin-5-yl]amino]-3-Pyridinecarboxamid; 6-[[5-fluoro-2-[3,4,5-trimethoxyphenyl)amino]-4-pyrimidinyl]amino]-2,2-dimethyl-2H-Pyrido[3,2-b]-1,4-oxazin-3(4H)-on; N-[3-bromo-7-(4-methoxyphenyl)-1,6-naphthyridin-5-yl]-1,3-Propanediamin, 7-(4-methoxyphenyl)-N-methyl-1,6-Naphthyridin-5-amin; N-[7-(4-methoxyphenyl)-1,6-naphthyridin-5-yl]-1,3-Propanediamin; N-[7-(2-thienyl)-1,6-naphthyridin-5-yl-1,3-Propanediamin; N-[7-[4-(dimethylamino)phenyl]-1,6-naphthyridin-5-yl]-1,2-Ethanediamin; N-[7-(4-methoxyphenyl)-2-(trifluoromethyl)-1,6-naphthyridin-5-yl]- 1,3-Propanediamin; N-[7-(4-methoxyphenyl)-3-phenyl-1,6-naphthyridin-5-yl]-1,3-Propanediamin; N-(7-phenyl-1,6-naphthyridin-5-yl)-1,3-Propanediamin; N-[7-(3-fluorophenyl)-1,6-naphthyridin-5-yl]-1,3-Propanediamin; N-[7-(3-chlorophenyl)-1,6-naphthyridin-5-yl]-1,3-Propanediamin; N-[7-[3-(trifluoromethoxy)phenyl]-1,6-naphthyridin-5yl]-1,3-Propanediamin;N-[7-(4-fluorophenyl)-1,6-naphthyridin-5-yl]-1,3-Propanediamin; N-[7-(4-fluorophenyl)-1,6-naphthyridin-5-yl]-1,3-Propanediamin; N-[7-(4-chlorophenyl)-1,6-naphthyridin-5-yl]-1,3-Propanediamin; N-[7-(4'-methyl[1,1'-biphenyl]-4-yl)-1,6-naphthyridin-1,3-Propanediamin; N-[7-[4-(dimethylamino)phenyl]-1,6-naphthyridin-5-yl]-1,3-Propanediamin; N-[7-[4-(diethylamino)phenyl]-1,6-naphthyridin-5-yl]-1,3-Propanediamin; N-[7-[4-(4-morpholinyl)phenyl]-1,6-naphthyridin-5-yl]-1,3-Propanediamin; N-[7-[4-[[2-(dimethylamino)ethyl]methylamino]phenyl]-1,6-naphthyridin-5-yl]-1,3-Propanediamin; N-[7-(4-bromophenyl)-1,6-naphthyridin-5-yl]-1,3-Propanediamin; N-[7-(4-methylphenyl)-1,6-naphthyridin-5-yl]-1,3-Propanediamin; N-[7-[4-(methylthio)phenyl]-1,6-naphthyridin-5-yl]-1,3-Propanediamin; N-[7-[4-(1-methylethyl)phenyl]-1,6-naphthyridin-5-yl]-1,3-Propanediamin; 7-[4-(dimethylamino)phenyl]-N-methyl-1,6-Naphthyridin-5-amin; 7-[4-(dimethylamino)phenyl]-N,N-dimethyl-1,6-Naphthyridin-5-amin; N-[7-[4-(dimethylamino)phenyl]-1,6-naphthyridin-5-yl]-1,4-Butanediamin; N-[7-[4-(dimethylamino)phenyl]-1,6-naphthyridin-5-yl]-1,5-Pentanediamin; 3-[[7-[4-(dimethylamino)phenyl]-1,6-naphthyridin-5-yl]oxy]-1-Propanol; 4-[5-(4-aminobutoxy)-1,6-naphthyridin-7-yl]-N,N-dimethyl-Benzenamin; 4-[[7-[4-(dimethylamino)phenyl]-1,6-naphthyridin-5-yl]amino]-1-Butanol; N-[7-[4-(dimethylamino)phenyl]-1,6-naphthyridin-5-yl]-N-methyl-1,3-Propanediamin; N-[1-[4-(dimethylamino)phenyl]-1,6-naphthyridin-5-yl]-N'-methyl-1,3-Propanediamin; N-[7-[4-(dimethylamino)phenyl]-1,6-naphthyridin-5-yl]-N,N'-dimethyl-1,3-Propanediamin; 1-amino-3-[[7-[4-(dimethylamino)phenyl]-1,6-naphthyridin-5-yl]amino]-2-Propanol; N-[7-[4-(dimethylamino)phenyl]-1,6-naphthyridin-5-yl]-2,2-dimethyl-1,3-Propanediamin; 7-[4-(dimethylamino)phenyl]-N-(3-pyridinylmethyl)-1,6-Naphthyridin-5-amin; N-[(2-aminophenyl)methyl]-7-[4-(dimethylamino)phenyl]-1,6-Naphthyridin-5-amin; N-[7-[6-(dimethylamino)[1,1'-biphenyl]-3-yl]-1,6-naphthyridin-5-yl]-1,3-Propanediamin; N-[7-[3-chloro-4-(diethylamino)phenyl]-1,6-naphthyridin-5-yl]-1,3-Propanediamin; N-[7-[4-(dimethylamino)-3-methoxyphenyl]-1,6-naphthyridin-5-yl]-1,3-Propanediamin; N-[7-[4-(diethylamino)phenyl]-3-methyl-1,6-naphthyridin-5-yl]-1,3-Propanediamin; N-[7-(3'-fluoro[1,1'-biphenyl]-3-yl)-1,6-naphthyridin-5-yl]-1,2-Ethanediamin, N-[7-(4-methoxyphenyl)-1,6-naphthyridin-5-yl]-1,6-Naphthyridine-1,3-Propanediamin; N,N'-bis(3-aminopropyl)-7-(4-methoxyphenyl)-2,5-diamin; N-[7-(4-methoxyphenyl)-2-(phenylmethoxy)-1,6-naphthyridin-5-yl]-1,6-Naphthyridine-1,3-Propanediamin; N5-(3-aminopropyl)-7-(4-methoxyphenyl)-N2-(phenytmethyl)-2,5-diamin; N-[7-(2-naphthalenyl)-1,6-naphthyridin-5-yl]-1,3-Propanediamin; N-[7-(2'-fluoro[1,1'-biphenyl]-4-yl)-1,6-naphthyridin-5-yl]-1,3-Propanediamin; N-[7-(3,4,5-trimethoxyphenyl)-1,6-naphthyridin-5-yl]-1,3-Propanediamin; N-[7-(3,4-dimethylphenyl)-1,6-naphthyridin-5-yl]-1,3-Propanediamin; 1-amino-3-[[7-(2-naphthalenyl)-1,6-naphthyridin-5-yl]amino]-2-Propanol; 1-amino-3-[[7-(2'-fluoro[1,1'-biphenyl]-4-yl)-1,6-naphthyridin-5-yl]amino]-2-Propanol; 1-amino-3-[[7-(4'-methoxy[1,1'-biphenyl]-4-yl)-1,6-naphthyridin-5-yl]amino]-2-Propanol; 1-amino-3-[[7-(3,4,5-trimethoxyphenyl)-1,6-naphthyridin-5-yl]amino]-2-Propanol; 1-amino-3-[[7-(4-bromophenyl)-1,6-naphthyridin-5-yl]amino]-2-Propanol; N-[7-(4'-methoxy[1,1'-biphenyl]-4-yl)-1,6-naphthyridin-5-yl]-2,2-dimethyl-1,3-Propanediamin; 1-[[7-[4-(dimethylamino)phenyl]-1,6-naphthyridin-5-yl]amino]-2-Propanol; 2-[[2-[[7-[4-(dimethylamino)phenyl]-1,6-naphthyridin-5-yl]amino]ethyl]thio]-Ethanol; 7-[4-(dimethylamino)phenyl]-N-(3-methyl-5-isoxazolyl)-1,6-Naphthyridin-5-amin; 7-[4-(dimethylamino)phenyl]-N-4-pyrimidinyl-1,6-Naphthyridin-5-amin; N-[7-[4-(dimethylamino)phenyl]-1,6-naphthyridin-5-yl]-1,3-Cyclohexanediamin; N,N-dimethyl-4-[5-(1-piperazinyl)-1,6-naphthyridin-7-yl]-Benzenamin; 4-[5-(2-methoxyethoxy)-1,6-naphthyridin-7-yl]-N,N-dimethyl-Benzenamin; 1-[7-[4-(dimethylamino)phenyl]-1,6-naphthyridin-5-yl]-4-Piperidinol; 1-[7-[4-(dimethylamino)phenyl]-1,6-naphthyridin-5-yl]-3-Pyrrolidinol; 7-[4-(dimethylamino)phenyl]-N-(2-furanylmethyl)-1,6-Naphthyridin-5-amin; 7-[4-(dimethylamino)phenyl]-N-[3-(1H-imidazol-1-yl)propyl]-1,6-Naphthyridin-5-amin; 1-[7-[4-(dimethylamino)phenyl]-1,6-naphthyridin-5-yl]-4-Piperidinecarboxamid; 1-(3-[[7-[4-(dimethylamino)phenyl]-1,6-naphthyridin-5-yl]amino]propyl]-2-Pyrrolidinon; N-[3'-[5-[(3-aminopropyl)amino]-1,6-naphthyridin-7-yl][1,1'-biphenyl]-3-yl]-Acetamid; N-[7-(4'-fluoro[1,1'-biphenyl]-4-yl)-1,6-naphthyridin-5-yl]-1,3-Propanediamin; N-[4'-[5-[(3-aminopropyl)amino]-1,6-naphthyridin-7-yl][1,1'-biphenyl]-3-yl]-Acetamid; N-[7-[4-(1,3-benzodioxol-5-yl)phenyl]-1,6-naphthyridin-5-yl]-1,3-Propanediamin; N-[7-[4-(2-thienyl)phenyl]-1,6-naphthyridin-5-yl]-1,3-Propanediamin; N-[7-[4-fluoro-3-(trifluoromethyl)phenyl]-1,6-naphthyridin-5-yl]-1,3-Propanediamin; N-[7-[4-(3-pyridinyl)phenyl]-1,6-naphthyridin-5-yl]-1,3-Propanediamin; N-[7-(1,3-benzodioxol-5-yl)-1,6-naphthyridin-5-yl]-1,3-Propanediamin; N-[7-(6-methoxy-2-naphthalenyl)-1,6-naphthyridin-5-yl]-1,3-Propanediamin; 7-[4-(dimethylamino)phenyl]-N-(4-pyridinylmethyl)-1,6-Naphthyridin-5-amin; 3-[[7-[4-(dimelhylamino)phenyl]-1,6-naphthyridin-5-yl]methylamino]-Propanenitril; 7-[4-(dimethylamino)phenyl]-N-[1-(phenylmethyl)-4-piperidinyl]-1,6-Naphthyridin-5-amin; N-[7-[4-(dimethylamino)phenyl]-1,6-naphthyridin-5-yl]-1,2-Cyclohexanediamin, N-[7-[4-(dimethylamino)phenyl]-1,6-naphthyridin-5-yl]-1,2-Cyclohexanediamine, (1R,2S)-rel-., N-[7-[4-(dimethylamino)phenyl]-1,6-naphthyridin-5-yl]-1,2-Benzenedimethanamine; N-[7-[4-(diethylamino)phenyl]-1,6-naphthyridin-5-yl]-1,4-Butanediamine; N-[7-[3',5'-bis(trifluoromethyl)[1,1'-biphenyl]-4-yl]-1,6-naphthyridin-5-yl]-,3-Propanediamine; N-[7-(3'-methoxy[1,1'-biphenyl]-4-yl)-1,6-naphthyridin-5-yl]-1,3-Propanediamine; N-[7-(3'-fluoro[1,1'-biphenyl]-4-yl)-1,6-naphthyridin-5-yl]-1,3-Propanediamine; 4-[[7-[4-(dimethylamino)phenyl]-1,6-naphthyridin-5-yl]oxy]-1-Butanol; N-[7-[4-(dimethylamino)phenyl]-1,6-naphthyridin-5-yl]- 1,4-Cyclohexanediamine; 7-[4-(dimethylamino)phenyl]-N-(2,2,6,6-tetramethyl-4-piperidinyl)-1,6-Naphthyridin-5-amine; N-[7-[3-bromo-4-(dimethylamino)phenyl]-1,6-naphthyridin-5-yl]-1,3-Propanediamine; N-[7-(1-methyl-1H-indol-5-yl)-1,6-naphthyridin-5-yl]-1,3-Propanediamine; N-[7-[3-(trifluoromethyl)phenyl]-1,6-naphthyridin-5-yl]-1,3-Propanediamine; N-[7-[4-(trifluoromethyl)phenyl]-1,6-naphthyridin-5-yl]-1,3-Propanediamine; N-[7-(3-bromo-4-methoxyphenyl)-1,6-naphthyridin-5-yl]-1,3-Propanediamine; N-[7-[4-[[3-(dimethylamino)propyl]methylamino]phenyl]-1,6-naphthyridin-5-yl]-1,4-Cyclohexanediamine; N-[7-[4-[[2-(dimethylamino)ethyl]methylamino]phenyl]-1,6-naphthyridin-5-yl]-1,4-Cyclohexanediamine; N-[7-[4-(dimethylamino)-3-methoxyphenyl]-1,6-naphthyridin-5-yl]-1,4-Cyclohexanediamine; N-[7-[4-(4-morpholinyl)phenyl]-1,6-naphthyridin-5-yl]-1,4-Cyclohexanediamine; N-[7-[3-bromo-4-(4-morpholinyl)phenyl]-1,6-naphthyridin-5-yl]-1,4-Cyclohexanediamine; 4-[[7-[4-[[2-(dimethylamino)ethyl]methylamino]phenyl]-1,6-naphthyridin-5-yl]oxy]-Cyclohexanol; N-[7-[3-bromo-4-(4-morpholinyl)phenyl]-1,6-naphthyridin-5-yl]-1,3-Propanediamine; N,N-dimethyl-4-[5-(4-methyl-1-piperazinyl)-1,6-naphthyridin-7-yl]-Benzenamine; 4-[[7-[4-[[3-(dimethylamino)propyl]methylamino]phenyl]-1,6-naphthyridin-5-yl]oxy]-Cyclohexanol; N-[7-[4-[[2-(dimethylamino)ethyl]methylamino]phenyl]-1,6-naphthyridin-5-yl]-1,4-Butanediamine; [3-[[5-[(3-aminopropyl)amino]-7-(4-methoxyphenyl)-1,6-naphthyridin-2-yl]amino]propyl]-Carbamic acid-1,1-dimethylethyl ester,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als MAP Kinase Inhibitoren gelangen hierbei vorzugsweise Verbindungen zur Anwendung, die ausgewählt sind aus der Gruppe bestehend aus:

### Bentamapimod (AS-602801), Doramapimod (BIRB-796),

5-Carbamoylindole (SD-169), 6-[(aminocarbonyl)(2,6-difluorophenyl)amino]-2-(2,4-difluorophenyl)-3-Pyridinecarboxamide (VX-702), alpha-[2-[[2-(3-pyridinyl)ethyl]amino]-4-pyrimidinyl]-2-Benzothiazoleacetonitrile (AS-601245), 9,12-Epoxy-1H-diindolo[1,2,3-fg:3',2',1'-kl]pyrrolo[3,4-i][1,6]benzodiazocine-10-Carboxylsäure (CEP-1347) und 4-[3-(4-chlorophenyl)-5-(1-methyl-4-piperidinyl)-1H-pyrazol-4-yl]-Pyrimidine (SC-409),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als iNOS-Inhibftoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, 5,6-dihydro-6-methyl-4H-1,3-Thiazin-2-amine (AMT), L-Canavanin, 2-Iminopiperidin, S-Isopropylisothioharnstoff, S-Methylisothioharnstoff, S-Ethylisothiohamstoff, S-Methyltiocitrullin, S-Ethylthiocitrullin, L-NA (N^{ω}-Nitro-L-arginin), L-NAME (N^{ω}-Nitro-L-argininmethylester), L-NMMA (N^{ω}-Monomethyl-L-arginin), L-NIO (N^{ω}-Iminoethyl-L-ornithin), L-NIL (N^{ω}-Iminoethyl-lysin), (S)-6-Acetimidoylamino-2-amino-Hexansäure (1*H*-tetrazol-5-yl)-amid (SC-51), N-[[3-(aminomethyl)phenyl]methyl]-Ethanimidamide (1400W), (S)-4-(2-Acetimidoylamino-ethylsulfanyl)-2-amino-buttersäure (GW274150), 2-[2-(4-Methoxy-pyridin-2-yl)-ethyl]-3*H*-imidazo[4,5-b]pyridin (BYK191023), 2-((R)-3-Amino-1-phenyl-propoxy)-4-chlor-5-fluorbenzonitril, 2-((1R,3S)-3-Amino-4-hydroxy-1-thiazol-5-yl-butylsulfanyl)-6-trifluoromethyl-nicotinonitril, 2-((1R,3S)-3-Amino-4-hydroxy-1-thiazol-5-yl-butylsulfanyl)-4-chlor-benzonitril, 2-((1R,3S)-3-Amino-4-hydroxy-1-thiazol-5-yl-butylsulfanyl)-5-chlor-benzonitril, (2S,4R)-2-Amino-4-(2-chlor-5-trifluoromethyl-phenylsulfanyl)-4-thiazol-5-yl-butan-1-ol, 2-((1R,3S)-3-Amino-4-hydroxy-1-thiazol-5-yl-butylsulfanyl)-5-chlor-nicotinonitril, 4-((S)-3-Amino-4-hydroxy-1-phenyl-butylsulfanyl)-6-methoxy-nicotinonitril, substituierte 3-Phenyl-3,4-dihydro-1-Isochinolinamin wie z.B. (1S,5S,6R)-7-Chlor-5-methyl-2-aza-bicyclo[4.1.0]hept-2-en-3-ylamin (ONO-1714), (4R,5R)-5-Ethyl-4-methyl-thiazolidin-2-ylideneamin, (4R,5R)-5-Ethyl-4-methyl-selenazolidin-2-ylideneamin, 4-Aminotetrahydrobiopterin, (E)-3-(4-Chlor-phenyl)-*N*-(1-{2-oxo-2-[4-(6-trifluormethyl-pyrimidin-4-yloxy)-piperidin-1-yl]-ethylcarbamoyl}-2-pyridin-2-yl-ethyl)-acrylamid (FR260330), 3-(2,4-Difluor-phenyl)-6-[2-(4-imidazol-1-ylmethyl-phenoxy)-ethoxy]-2-phenyl-pyridin (PPA250), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-S-glutathione, Estradiol 17-beta-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glykocholat, Glykolithocholsäure sulfat" Ibuprofen, Indomethacin, Indoprofen, Ketoprofen, Lithocholsäuresulfat sulphat, Methotrexat, ((*E*)-3-[[[3-[2-(7-Chloro-2-chinolinyl)ethenyl]phenyl]-[[3-dimethylamino)-3-oxopropyl]thio]methyl]thio]-propansäure), alpha-Naphthyl-beta-D-glucuronid, Nitrobenzyl mercaptopurine ribosid, Probenecid, Sildenafil, Sulfinpyrazone, Taurochenodeoxycholate, Taurocholat, Taurodeoxycholat, Taurolithocholat, Topotecan, Trequinsin, Zaprinast und Dipyridamol, gegebenenfalls in Form ihrer Racemate, Enantiomere, Diastereomere und ihrer pharmakologisch verträglichen Säure-Additionssalze und Hydrate.

Als Leukotriene Biosynthese Inhibitoren wie zum Beispiel aus der Gruppe der 5-Lipoxygenase (5-LO) Inhibitoren, cPLA2 Inhibitoren, Leukotriene A4 hydrolase Inhibitoren oder FLAP Inhibitoren, gelangen hierbei vorzugsweise Verbindungen zur Anwendung, die ausgewählt sind aus der Gruppe bestehend aus Zileuton, Tipelukast, Licofelone, Darapladib,
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Nicht-steroidale antientzündliche Agenzien (NSAIDs) gelangen hierbei vorzugsweise Verbindungen zur Anwendung, die ausgewählt sind aus der Gruppe bestehend aus Piroxicam, Diclofenac, Naproxen, Flurbiprofen, Fenoprofen, Ketoprofen, Ibuprofen, Nimesulide, Indomethacin, Sulindac, Azapropazone, Phenylbutazone, Aspirin; Meloxicam, Celecoxib, Rofecoxib, Valdecoxib, Lumarocoxib, Parecoxib, Tenoxicam und Etoricoxib, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als CRTH2 Antagonisten gelangen hierbei vorzugsweise Verbindungen zur Anwendung, die ausgewählt sind aus der Gruppe bestehend aus Ramatroban und Laropiprant, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als DP1-Rezeptor Modulatoren gelangen hierbei vorzugsweise Verbindungen zur Anwendung, die ausgewählt sind aus der Gruppe bestehend aus 7-[(1R,2R,3S,5S)-2-[[(5-hydroxybenzo[b]thien-3-yl)carbonyl]amino]-6,6-dimethylbicyclo[3.1.1]hept-3-yl]-, (5Z)-5-Heptenoic acid (S-5751), Laropiprant, und 2-[[4-[(1R,2S,3R,SR)-5-chloro-2-[(3S)-3-cyclohexyl-3-hydroxy-1-propyn-1-yl]-3-hydroxycyclopentyl]butyl]thio]-Essigsäure (TS-002), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Thromboxane Rezeptor Antagonist gelangen hierbei vorzugsweise Verbindungen zur Anwendung, die ausgewählt sind aus der Gruppe bestehend aus Seratrodast, N-[[(1,1-dimethylethyl)amino]carbonyl]-2-[(4-methylphenyl)amino]-5-nitro-Benzenesulfonamide (BM-573), (+/-)-sodium[2-(4-chlorophenylsulfonylarninomethyl)- indan-5-yl]acetate monohydrate (Z-335) und 2-[[[4-[[(4-chlorophenyl)sulfonyl]amino]butyl][[3-[[4-(1-methylethyl)-2-thiazolyl]methoxy]phenyl]methyl]amino]sulfonyl]-Benzoesäure (KP-496) gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Chemokine Rezeptor Antagonisten gelangen hierbei vorzugsweise Verbindungen zur Anwendung, die ausgewählt sind aus der Gruppe bestehend aus N-[5-chloro-2-[2-[(2R)-4-[(4-fluorophenyl)methyl]-2-methyl-1-piperazinyl]-2-oxoethoxy]phenyl]-Harnstoff hydrochloride (1:1) (BX-471), 2, N-[(1S,2S,4R)-4-(aminocarbonyl)-1-[(3-fluorophenyl)methyl]-2,7-dihydroxy-7-methyloctyl]- -Quinoxalinecarboxamide (CP-481715), (4,6-dimethyl-5-pyrimidinyl)[4-[(3S)-4-[(1R)-2-methoxy-1-[4-(trifluoromethyl)phenyl]ethyl]-3-methyl-1-piperazinyl]-4-methyl-1-piperidinyl]-Methanone (Sch-417690), 2-hydroxy-N,N-dimethyl-3-[[2-[[(1R)-1-(5-methyl-2-furanyl)propyl]amino]-3,4-dioxo-1-cyclobuten-1-yl]amino]-Benzamide (SCH-527123) und 1,4,8,11-Tetraazacyclotetradecane, 1,1'-[1,4-phenylenebis(methylene)]bis-, hydrochloride (1:8) (AMD-3100), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Neurokinin (NK1 oder NK2) Antagonisten gelangen hierbei vorzugsweise Verbindungen zur Anwendung, die ausgewählt sind aus der Gruppe bestehend aus: Saredutant, Nepadutant und Figopitant gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Sphingosinel-phosphat Rezeptor Modulatoren gelangen hierbei vorzugsweise Verbindungen zur Anwendung wie z.B. sonepcizumab, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Mucoregulatoren gelangen hierbei vorzugsweise Verbindungen zur Anwendung, die ausgewählt sind aus der Gruppe bestehend aus: 3-[2-oxo-2-[2-[[3-(trifluoromethyl)phenyl]amino]-3-pyridinyl]ethyl]-1(3H)-Isobenzofuranone (MSI-2216), Erdosteine, Fluorovent, Talniflumate, fudosteine, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als PPAR gamma Agonist gelangen hierbei vorzugsweise Verbindungen zur Anwendung, die ausgewählt sind aus der Gruppe bestehend aus: Rosiglitazone, Ciglitazone, Pioglitazone und N-[2-[2-[(3-fluorophenyl)imino]-4-[4-(4-morpholinyl)phenyl]-3(2H)-thiazolyl]ethyl]-N'-methyl-Harnstoff (SMP-028), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Rho Kinase Inhibitoren gelangen hierbei vorzugsweise Verbindungen zur Anwendung, wie z.B. Fasudil, gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Als Adenosine Rezeptor Modulatoren gelangen hierbei vorzugsweise Verbindungen zur Anwendung, die ausgewählt sind aus der Gruppe bestehend aus 4-(3,4-dichlorophenyl)-5-(4-pyridinyl)-2-Thiazolamine (CGH-2466), 3-ethyl-3,9-dihydro-1-propyl-8-[1-[[3-(trifluoromethyl)phenyl]methyl]-1H-pyrazol-4-yl]-1H-Purine-2,6-dione (CVT-6883), N-(4-cyanophenyl)-2-[4-(2,3,6,9-tetrahydro-2,6-dioxo-1,3-dipropyl-1H-purin-8-yl)phenoxyl-Acetamide (MRS-1754), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Bradykinin Rezeptor Antagonisten gelangen hierbei vorzugsweise Verbindungen zur Anwendung, die ausgewählt sind aus der Gruppe bestehend aus Icatibant und 1-Piperazinepentanaminium, delta-amino-4-[[4-[[[2,4-dichloro-3-[[(2,4-dimethyl-8-chinolinyl)oxy]methyl]phenyl]sulfonyl]amino]tetrahydro-2H-pyran-4-yl]carbonyl]-N,N,N-trimethyl-ε-oxo-, chloride, hydrochloride (1:1:1), (deltaS)- (MEN-16132), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Endothelin Antagonisten gelangen hierbei vorzugsweise Verbindungen zur Anwendung, die ausgewählt sind aus der Gruppe bestehend aus Actelion-1, Ambrisentan, Sitaxsentan, N-(2-acetyl-4,6-dimethylphenyl)-3-[[(4-chloro-3-methyl-5-isoxazolyl)amino]sulfonyl]-2-Thiophenecarboxamide (TBC-3214) und Bosentan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Interleukin 1-beta converting enzyme (ICE) Inhibitoren gelangen hierbei vorzugsweise Verbindungen zur Anwendung, die ausgewählt sind aus der Gruppe bestehend aus Pralnacasan und N-(4-amino-3-chlorobenzoyl)-3-methyl-L-valyl-N-[(2R,3S)-2-ethoxytetrahydro-5-oxo-3-furanyl]-L-Prolinamide (=VX-765), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Toll-like Rezeptor (TLR) Modulatoren gelangen hierbei vorzugsweise Verbindungen zur Anwendung, die ausgewählt sind aus der Gruppe bestehend aus Resiquimod, Heplisav, Resatorvid (TAK-242), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als HMG-CoA Reductase Inhibitoren gelangen hierbei vorzugsweise Verbindungen zur Anwendung, die ausgewählt sind aus der Gruppe bestehend aus Lovastatin, Simvastatin, Pravastatin, Fluvastatin und Avorvastatin, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als VLA-4 Antagonisten gelangen hierbei vorzugsweise Verbindungen zur Anwendung, die ausgewählt sind aus der Gruppe bestehend aus Natalizumab, valategrast, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als SHIP Agonisten gelangen hierbei vorzugsweise Verbindungen zur Anwendung, die ausgewählt sind aus der Gruppe bestehend aus 2,3,4,4a,5,6,6a,11,11a,11b-decahydro-4,4,6a,7,11b-pentamethyl-, (4aS,6aR,11aR,11bS)-1H-Benzo[a]fluoren-9-ol (AQX-MN100) und MN-106, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als anti-TNF-Antikörper gelangen hierbei vorzugsweise Verbindungen zur Anwendung, die ausgewählt sind aus der Gruppe bestehend aus Infliximab, Adalimumab, Golimumab. CytoFab und Etanercept.

Als Substanzen gegen Schwellungen der Atemwege gelangen hierbei vorzugsweise Verbindungen zur Anwendung, die ausgewählt sind aus der Gruppe bestehend aus Phenylephrine, Phenylpropanolamine, Pseudophedrine, Oxymetazoline, Epinephrine, Naphazoline, Xylometazoline, Propylhexedrine und Llevo-desoxyephedrine, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen gegen Husten gelangen hierbei vorzugsweise Verbindungen zur Anwendung, die ausgewählt sind aus der Gruppe bestehend aus Hydrocodone, Caramiphen, Carbetapentane und Dextramethorphan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter Lipoxin A4 Derivative gelangen hierbei vorzugsweise Verbindungen zur Anwendung, die ausgewählt sind aus der Gruppe bestehend aus 7,9,11,13-Eicosatetraenoic acid, 5,6,15-trihydroxy-, (5S,6R,7E,9E,11Z,13E,15R)- (15-epi-lipoxin a4), 7,9,11,13-Eicosatetraenoic acid, 16-(4-iluorophenoxy)-5,6,15-trihydroxy-, (5S,6R,7E,9E,11Z,13E,15S)-(ATL-1), aspirin-triggered lipoxin A(4) and analogs, protectin D1 (4,7,11,13,15,19-Docosahexaenoic acid, 10,17-dihydroxy-, (4Z,7Z,10R,11E,13E,15Z,17S,19Z)-, Resolvin E1 (6,8,10,14,16-Eicosapentaenoic acid, 5,12,18-trihydroxy-, (5S,6Z,8E,10E,12R,14Z,16E,18R)- ) and benzo-lipoxin A4 analogs, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter FPRL1-Modulatoren gelangen hierbei vorzugsweise Verbindungen zur Anwendung, wie z.B. 5(S),6(R), 7-trihydroxyheptanoic acid methyl ester, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter PI3 Kinase Antagonisten gelangen hierbei vorzugsweise Verbindungen zur Anwendung, die ausgewählt sind aus der Gruppe bestehend aus 5-(Quinoxalin-6-ylmethylene)thiazolidine-2,4-dione (AS-605240), 2-[(6-amino-9H-purin-9-yl)methyl]-5-methyl-3-(2-methylphenyl)-4(3H)-Quinazolinone (C-87114), and 2-Methyl-2-[4-[3-methyl-2-oxo-8-(chinolin-3-yl)-2,3-dihydroimidazo[4,5-c]chinolin-1-yl]phenyl]propionitrile (BEZ-235), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter CCR5 Antagonisten gelangen hierbei vorzugsweise Verbindungen zur Anwendung, die ausgewählt sind aus der Gruppe bestehend aus maraviroc (4,4-difluoro-N-[(1S)-3-[(3-exo)-3-[3-methyl-5-(1-methylethyl)-4H-1,2,4-triazol-4-yl]-8-azabicyclo[3.2.1]oct-8-yl]-1-phenylpropyl]-Cyclohexancarboxamid), CCR5mAb004, vicriviroc ((4,6-dimethyl-5-pyrimidinyl)[4-[(3S)-4-[(1R)-2-methoxy-1-[4-(trifluoromethyl)phenyl]ethyl]-3-methyl-1-piperazinyl]-4-methyl-1-piperidinyl]-Methanon)
und nifeviroc (N-[1-[[(3S,4R)-1-(cyclopentylcarbonyl)-4-hydroxy-4-phenyl-3-pyrrolidinyl]methyl]-4-piperidinyl]-N-2-propen-1-yl- (4-nitrophenyl)methyl ester-Carbaminsäure),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter CXCR1 or CXCR2 Antagonisten gelangen hierbei vorzugsweise Verbindungen zur Anwendung, wie z.B. 3-[[3-[(Dimethylamino)carbonyl]-2-hydroxyphenyl]amino]-4-[[(R)-1-(5-methylfuran-2-yl)propyl]amino]cyclobut-3-ene-1,2-dione (SCH-527123),
gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Erfindungsgemäß bevorzugt sind die Säureadditionssalze der oben genannten MAP Kinase Inhibitoren, iNOS-Inhibitoren, MRP4-Inhibitoren, Leukotriene Biosynthese Inhibitoren, Nichtsteroidalen antientzündlichen Agenzien (NSAIDs), CRTH2 Antagonisten, DP1-Rezeptor Modulatoren, Thromboxane Rezeptor Antagonisten, Chemokine Rezeptor Antagonisten, Neurokinin (NK1 oder NK2) Antagonisten, Sphingosinel-phosphat Rezeptor Modulatoren, Mucoregulatoren, PPAR gamma Agonisten, Rho Kinase Inhibitoren, Adenosine Rezeptor Modulatoren, Bradykinin Rezeptor Antagonisten. Endothelin Antagonisten, Interleukin 1-beta converting enzyme (ICE) Inhibitoren, Toll-like Rezeptoren (TLR) Modulatoren, HMG-CoA Reductase Inhibitoren, VLA-4 Antagonisten, SHIP Agonisten, anti-TNF-Antikörper, Substanzen gegen Schwellungen der Atemwege, Substanzen gegen Husten, Lipoxin A4 Derivative, PI3 Kinase Antagonisten, FPRL1-Modulatoren, CCR5 Antagonisten ,CXCR1- oder CXCR2-Antagonisten ebenfalls ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

### Formulierungen

Die erfindungsgemäßen Verbindungen können oral, transdermal, inhalativ, parenteral oder sublingual verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 0.1 und 5000, vorzugsweise zwischen 1 und 500, besonders bevorzugt zwischen 5-300 mg/Dosis, bei intravenöser, subkutaner oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern, ethanolischen oder wässrigen Lösungen bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmittein, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Bei der pharmazeutischen Anwendung werden die erfindungsgemäßen Verbindungen in der Regel bei warmblütigen Wirbeltieren, insbesondere beim Menschen, in Dosierungen von 0.01-100 mg/kg Körpergewicht, vorzugsweise bei 0.1-15 mg/kg verwendet. Zur Verabreichung können diese beispielsweise mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragées, Kapseln, Pulver, Suspensionen, Lösungen, Sprays oder Zäpfchen eingearbeitet werden.

Die nachfolgenden Beispiele illustrieren die vorliegende Erfindung ohne sie jedoch in ihrem Umfang zu beschränken:

### Pharmazeutische Formulierungsbeispiele

### A) Dragées mit 75 mg Wirksubstanz

### Zusammensetzung:

| 1 | Dragéekern enthält: | |
|---|---|---|
| | Wirksubstanz | 75.0mg |
| | Calciumphosphat | 93.0 mg |
| | Maisstärke | 35.5 mg |
| | Polyvinylpyrrolidon | 10.0 mg |
| | Hydroxypropylmethylcellulose | 15.0 mg |
| | Magnesiumstearat | 1.5 mg |
| | | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kemgewicht: | 230 mg |
| Stempel: | 9 mm, gewölbt |

Die so hergestellten Dragéekeme werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht: | 245 mg. |

### B) Tabletten mit 100 mg Wirksubstanz

### Zusammensetzung:

| 1 | Tablette enthält: | |
|---|---|---|
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu

### Tabletten verarbeitet.

| | |
|---|---|
| Tablettengewicht: | 220 mg |
| Durchmesser: | 10 mm, biplan mit beidseitiger Facette und einseitiger Teilkerbe. |

### C) Tabletten mit 150 mg Wirksubstanz

### Zusammensetzung:

| 1 | Tablette enthält: | |
|---|---|---|
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### D) Hartgelatine-Kapseln mit 150 mg Wirksubstanz

### Zusammensetzung:

| 1 Kapsel enthält: | |
|---|---|
| Wirkstoff | 150.0 mg |
| Maisstärke getr. ca. | 180.0 mg |
| Milchzucker pulv. ca. | 87.0 mg |
| Magnesiumstearat | 3.0 mg |
| ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.

Die Endmischung wird in Hartgelatine-Kapseln der Größe 1 abgefüllt.
Kapselfüllung: ca. 320 mg
Kapselhülle: Hartgelatine-Kapsel Größe 1.

### E) Suppositorien mit 150 mg Wirksubstanz

### Zusammensetzung:

| 1 Zäpfchen enthält: | |
|---|---|
| Wirkstoff | 150.0 mg |
| Polyäthylenglykol 1500 | 550.0 mg |
| Polyäthylenglykol 6000 | 460.0 mg |
| Polyoxyäthylensorbitanmonostearat | 840.0 mg |
| | 2000.0 mg |

### Herstellung,

Nach dem Aufschmelzen der Suppositorienmasse wird der Wirkstoff darin homogen verteilt und die Schmelze in vorgekühlte Formen gegossen.

### F) Suspension mit 50 mg Wirksubstanz

### Zusammensetzung:

| 100 ml Suspension enthalten: | |
|---|---|
| Wirkstoff | 1.00 g |
| Carboxymethylcellulose-Na-Salz | 0.10 g |
| p-Hydroxybenzoesäuremethylester | 0.05 g |
| p-Hydroxybenzoesäurepropylester | 0.01 g |
| Rohrzucker | 10.00 g |
| Glycerin | 5.00 g |
| Sorbitlösung 70%ig | 20.00 g |
| Aroma | 0.30 g |
| Wasser dest. ad | 100.00 ml |

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### G) Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### H) Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### I) Kapseln zur Pulverinhalation mit 5 mg Wirksubstanz

### 1 Kapsel enthält:

| | |
|---|---|
| Wirksubstanz | 5.0 mg |
| Lactose für Inhalationszwecke | 15.0 mg |
| | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

### J) Inhalationslösung für Handvernebler mit 2.5 mg Wirksubstanz

### 1 Hub enthält:

| | |
|---|---|
| Wirksubstanz | 2.500 mg |
| Benzalkoniumchlorid | 0.001 mg |
| 1N-Salzsäure q.s. | |
| Ethanol/Wasser (50/50)ad | 15.000 mg |

### Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.
Füllmasse des Behälters: 4.5 g

## Patentansprüche

1. Verbindungen der allgemeinen Formel (I) 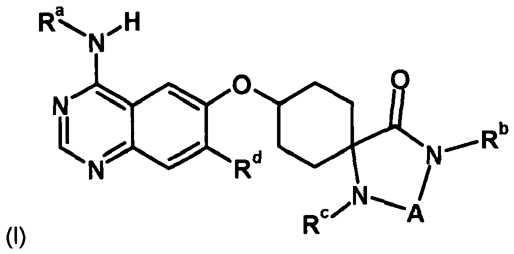 **dadurch gekennzeichnet, dass**
**R^{a}** eine Phenyl- oder 1-Phenylethyl-Gruppe, in denen der Phenylkern jeweils durch die Reste R¹ bis R³ substituiert ist, wobei
**R¹ und R²** gleich oder verschieden, Wasserstoff oder einen Rest ausgewählt aus der Gruppe bestehend aus F, Cl, Br, I, OCH₂F, OCHF₂, OCF₃, CH₂F, CHF₂, CF₃, CN, NO₂, NH₂ und OH,
oder
einen Rest ausgewählt aus der Gruppe bestehend aus C₁₋₄-Alkyl, C₁₋₄-Alkyl-O-, C₂₋₃-Alkenyl, C₂₋₃-Alkinyl, Phenyl, Phenyl-O-, Phenyl-C₁₋₃-alkyl-, Phenyl-C₁₋₃-alkyl-O-, Heteroaryl, Heteroaryl-O-, Heteroaryl-C₁₋₃-alkyl-, Heteroaryl-C₁₋₃-alkyl-O-, wobei die vorstehend genannten Phenylgruppen durch Reste R⁵, mono- oder disubstituiert sind,
und
**R³** Wasserstoff,
oder
einen Rest ausgewählt aus der Gruppe bestehend aus F, Cl, Br und CH₃,
**R^{b}** Wasserstoff, oder einen Rest, gegebenenfalls substituiert, ausgewählt aus der Gruppe bestehend aus C₁₋₆-Alkyl, C₃₋₆-Cycloalkyl- und C₃₋₆-Cycloalkyl-C₁₋₃-alkyl,
**R^{c}** Wasserstoff, oder einen Rest, gegebenenfalls substituiert, ausgewählt aus der Gruppe bestehend aus C₁₋₆-Alkyl, C₃₋₆-Cycloalkyl-, C₃₋₆-Cycloalkyl-C₁₋₃-alkyl, C₁₋₆-Alkyl-C0-, C₃₋₆-Cycloalkyl-CO-, C₃₋₆-Cycloalkyl-C₁₋₃-alkyl-CO-, C₁₋₆-Alkyl-SO₂-, C₃₋₆-Cycloalkyl-SO₂-, C₃-₆-Cycloalkyl-C₁₋₃-alkyl-SO₂-, Phenyl-CO- und Phenyl-SO₂-,
**R^{d}** Wasserstoff oder
einen Rest ausgewählt aus der Gruppe bestehend aus F, Cl, Br, I, OH, C₁₋₄-Alkyl, C₁₋₄-Alkyl-O-, mit 1 bis 3 Fluoratomen substituiertes C₁₋₂-Alkyl-O-, C₃₋₇-Cycloalkyl-O-, C₃₋₇-Cycloalkyl-C₁₋₄-alkyl-O-, Tetrahydrofuran-3-yl-O-, Tetrahydropyran-3-yl-O-, Tetrahydro-pyran-4-yl-O-, Tetrahydrofuranyl-C₁₋₄-alkyl-O- und Tetrahydropyranyl-C₁₋₄-alkyl-O-,
oder
R⁴-C₁₋₄-alkyl-, wobei die Verknüpfung der Reste R⁴ über jedes C-Atom des Alkylrestes erfolgen kann,
oder
R⁴-C₂₋₄-alkyl-O-, wobei der Rest R⁴ durch mindestens 2 C-Atome von demSauerstoffatom getrennt ist,
oder
einen Rest ausgewählt aus der Gruppe bestehend aus Pyrrolidin-2-yl-C₁₋₄-alkyl-O-, Pyrrolidin-3-yl-C₁₋₄-alkyl-O-, Piperidin-2-yl-C₁₋₄-alkyl-O-, Piperidin-3-yl-C₁₋₄-alkyl-O-, Piperidin-4-yl-C₁₋₄-alkyl-O-, Azepan-2-yl-C₁₋₄-alkyl-O-, Azepan-3-yl-C₁₋₄-alkyl-O-, Azepan-4-yl-C₁₋₄-alkyl-O-, Morpholin-2-yl-C₁₋₄-alkyl-O-, Morpholin-3-yl-C₁₋₄-alkyl-O-, 1-(C₁₋₃-Alkyl)-pyrrolidin-2-yl-C₁₋₄-alkyl-O-, 1-(C₁₋₃-Alkyl)-pyrrolidin-3-yl-C₁₋₄-alkyl-O-, 1-(C₁₋₃-Alkyl)-piperidin-2-yl-C₁₋₄-alkyl-O-, 1-(C₁₋₃-Alkyl)-piperidin-3-yl-C₁₋₄-alkyl-O-, 1-(C₁₋₃-Alkyl)-piperidin-4-yl-C₁₋₄-alkyl-O-, 1-(C₁₋₃-Alkyl)-azepan-2-yl-C₁₋₄-alkyl-O-, 1-(C₁₋₃-Alkyl)-azepan-3-yl-C₁₋₄-alkyl-O-, 1-(C₁₋₃-Alkyl)-azepan-4-yl-C₁₋₄-alkyl-O-, 4-(C₁₋₃-Alkyl)-morpholin-2-yl-C₁₋₄-alkyl-O- und 4-(C₁₋₃-Alkyl)-morpholin-3-yl-C₁₋₄-alkyl-O-,
wobei
**R⁴** einen Rest, gleich oder verschieden, ausgewählt aus der Gruppe bestehend aus OH, C₁₋₃-Alkyl-O-, C₃₋₆-Cycloalkyl-O-, NH₂, C₁₋₃-Alkyl-NH-, (C₁₋₃-Alkyl)₂N-, (2-Methoxyethyl)₂N-, Pyrrolidin-1-yl-, Piperidin-1-yl-, Azepan-1-yl-, Morpholin-4-yl-, 1,4-Oxazepan-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo[3.2.1]oct-3-yl-, Piperazin-1-yl-, 4-(C₁₋₃-Alkyl)-piperazin-1-yl-, 1,4-Diazepan-1-yl-, 4-(C₁₋₃-Alkyl)-1,4-diazepan-1-yl, HCO-NH-, C₁₋₄-Alkyl-CO-NH-, C₁₋₃-Alkyl-O-C₁₋₃-alkyl-CO-NH-, C₁₋₄-Alkyl-O-CO-NH-, H₂NCONH-, C₁₋₃-Alkyl-NH-CO-NH-, (C₁₋₃-Alkyl)₂N-CONH-, Pyrrolidin-1-yl-CO-NH-, Piperidin-1-yl-CO-NH -, Piperazin-1-yl-CO-NH-, 4-(C₁₋₃-Alkyl)-piperazin-1-yl-CO-NH -, Morpholin-4-yl-CO-NH- und C₁₋₄-Alkyl-SO₂-NH-,
wobei die bei der Definition des Restes R^{d} vorstehend genannten Pyrrolidinyl-, Piperidinyl-, Azepan-1-yl-, Piperazinyl-, 1,4-Diazepan-1-yl-, Morpholinyl- und 1,4-Oxazepan-4-yl-Gruppen jeweils zusätzlich durch eine oder zwei C₁₋₃-Alkylgruppen substituiert sein können,
und
wobei die bei der Definition des Restes R^{d} vorstehend genannten Phenylgruppen durch Reste R⁵ mono- oder di- substituiert sind, wobei
**R⁵** Wasserstoff, oder einen Rest, gleich oder verschieden, ausgewählt aus der Gruppe bestehend aus F, Cl, Br, I, OH, CN, C₁₋₃-Alkyl-, C₁₋₃-Alkyl-O-, CHF₂, CF₃, -O-CHF₂ und -O-CF₃,
und
soweit nichts anderes erwähnt wurde, die vorstehend erwähnten Alkylgruppen geradkettig oder verzweigt sein können,
**A** -CO- oder -C₁-C₃-alkylen-, wobei der -C₁-C₃-alkylen- Rest durch einen Rest R⁶ 1-, 2-, 3- oder 4-fach substituiert sein kann,
und
**R⁶** gleich oder verschieden, Wasserstoff, oder einen Rest ausgewählt aus der Gruppe bestehend aus OH, C₁-C₄-Alkyl und -O-C₁-C₄-Alkyl,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

2. Verbindungen nach Anspruch 1,
**dadurch gekennzeichnet, dass**
**R^{a}** einen Rest ausgewählt aus der Gruppe bestehend aus 3-Chlor-2-fluor-phenyl-, 3-Chlor-4-fluor-phenyl-, 5-Chlor-2-fluor-phenyl-, 2-Fluor-3-methyl-phenyl-, 2-Fluor-5-methylphenyl-, 4-Fluor-3-methyl-phenyl- und 3-Chlor-2-methyl-phenyl-Gruppe,
**R^{b} und R^{c}** gleich oder verschieden, Wasserstoff oder C₁₋₃-Alkyl,
**R^{d}** C₁₋₃-Alkyl-O-,
soweit nichts anderes erwähnt wurde, die vorstehend erwähnten Alkylgruppen geradkettig oder verzweigt sein können,
**A** -CH₂CH₂-, wobei der -CH₂CH₂- Rest durch eine 1 oder 2 Methylgruppen substituiert sein kann,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

3. Verbindung 1 nach Anspruch 1 oder 2, 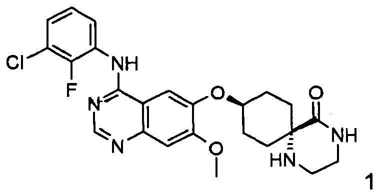 gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,

4. Verbindung 1(1) nach Anspruch 1 oder 2, 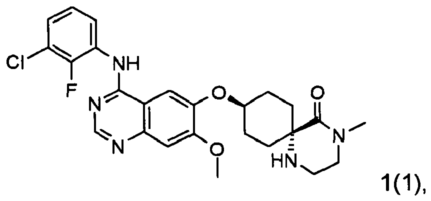 gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,

5. Verbindung 1(2) nach Anspruch 1 oder 2, 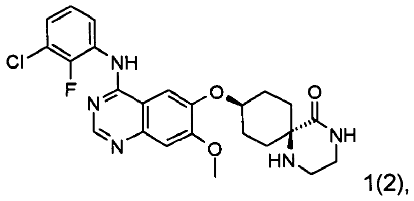 gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,

6. Verbindung 2 nach Anspruch 1 oder 2, 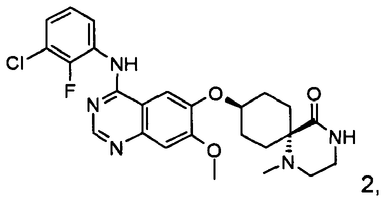 gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,

7. Verbindung 2(1) nach Anspruch 1 oder 2, 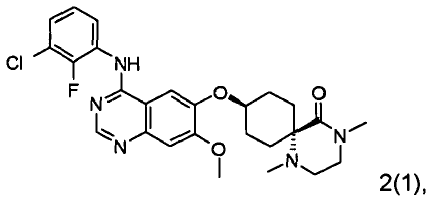 gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,

8. Verbindung 3 nach Anspruch 1 oder 2, 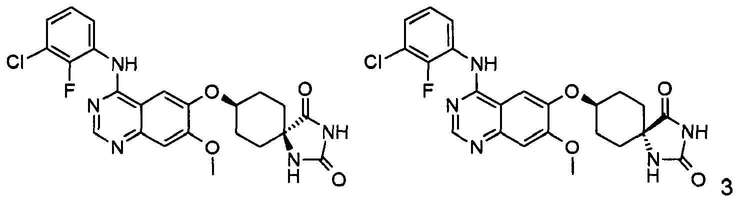 gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,

9. Verbindungen der Formel (I) nach einem der Ansprüche 1 bis 8 zur Verwendung als Arzneimittel.

10. Verbindungen der Formel (I) nach einem der Ansprüche 1 bis 8, zur Behandlung entzündlicher oder allergischer Erkrankungen der Atemwege.

11. Verbindungen nach Anspruch 10 zur Behandlung einer Erkrankung, die ausgewählt ist aus der Gruppe bestehend aus Chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen, Alveolitis, hyperreaktiver Atemwege, Nasenpolypen, Lungenödemen, Pneumonitis aufgrund unterschiedlicher Genese wie Strahlen-induziert oder durch Aspiration oder infektiöse, Kollagenosen wie Lupus eryth, systemische Sklerodermie, Sarkoidose und M. Boeck.

12. Verbindungen der Formel (I) nach einem der Ansprüche 1 bis 8 zur Behandlung entzündlicher oder allergischer Krankheitszustände, bei denen Autoimmun-Reaktionen beteiligt sind.

13. Verbindungen der Formel (I) nach einem der Ansprüche 1 bis 8 zur Behandlung von benignen oder malignen Tumoren.

14. Pharmazeutische Formulierung enthaltend eine Verbindung der Formel (I) gemäß einem der Ansprüche 1 oder 8.

15. Oral applizierbare pharmazeutische Formulierung nach Anspruch 14 enthaltend eine Verbindung der Formel (I) gemäß Anspruch 1 bis 8.

16. Arzneimittelkombinationen, die neben einer oder mehrerer Verbindungen der Formel (I) gemäß einem der Ansprüche 1 bis 8 als weiteren Wirkstoff einen oder mehrere Verbindungen enthalten, die ausgewählt sind aus den Klassen der Betamimetika, Anticholinergika, Corticosteroiden, weitere PDE4-Inhibitoren, LTD4-Antagonisten, EGFR-Hemmern, Dopamin-Agonisten, H1-Antihistaminika, PAF-Antagonisten und PI3-Kinase Inhibitoren oder zwei- oder dreifach Kombinationen davon.

## Claims

1. Compounds of general formula (I) 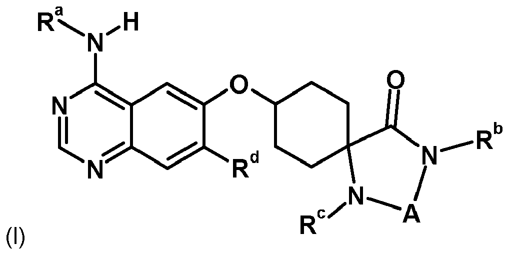 **characterised in that**
**R^{a}** denotes a phenyl or 1-phenylethyl group, wherein the phenyl nucleus is substituted in each case by the groups R¹ to R³, wherein
**R¹ and R²** which may be identical or different, denote hydrogen or a group selected from among F, Cl, Br, I, OCH₂F, OCHF₂, OCF₃, CH₂F, CHF₂, CF₃, CN, NO₂, NH₂ and OH,
or
a group selected from among C₁₋₄-alkyl, C₁₋₄-alkyl-O-, C₂₋₃-alkenyl, C₂₋₃-alkynyl, phenyl, phenyl-O-, phenyl-C₁₋₃-alkyl-, phenyl-C₁₋₃-alkyl-O-, heteroaryl, heteroaryl-O-, heteroaryl-C₁₋₃-alkyl-, heteroaryl-C₁₋₃-alkyl-O-, wherein the above-mentioned phenyl groups are mono- or disubstituted by groups R⁵,
and
**R³** denotes hydrogen, or a group selected from among F, Cl, Br and CH₃,
**R^{b}** denotes hydrogen, or an optionally substituted group selected from among C₁₋₆-alkyl, C₃₋₆-cycloalkyl- and C₃₋₆-cycloalkyl-C₁₋₃-alkyl,
**R^{c}** denotes hydrogen, or an optionally substituted group selected from among C₁₋₆-alkyl, C₃₋₆-cycloalkyl-, C₃₋₆-cydoalkyl-C₁₋₃-alkyl, C₁₋₆-alkyl-CO-, C₃₋₆-cycloalkyl-CO-, C₃₋₆-cycloalkyl-C₁₋₃-alkyl-CO-, C₁₋₆-alkyl-SO₂-, C₃₋₆-cycloalkyl-SO₂-, C₃₋₆-cycloalkyl-C₁₋₃-alkyl-SO₂-, phenyl-CO- and phenyl-SO₂-,
**R^{d}** denotes hydrogen or
a group selected from among
F, Cl, Br, I, OH, C₁₋₄-alkyl, C₁₋₄-alkyl-O-, C₁₋₂-alkyl-O- substituted by 1 to 3 fluorine atoms, C₃₋₇-cycloalkyl-O-, C₃₋₇-cycloalkyl-C₁₋₄-alkyl-O-, tetrahydrofuran-3-yl-O-, tetrahydropyran-3-yl-O-, tetrahydro-pyran-4-yl-O-, tetrahydrofuranyl-C₁₋₄-alkyl-O- and tetrahydropyranyl-C₁₋₄-alkyl-O-,
or
R⁴-C₁₋₄-alkyl-, wherein the linking of the groups R⁴ may take place via each C atom of the alkyl group,
or
R⁴-C₂₋₄-alkyl-O-, wherein the group R⁴ is separated from the oxygen atom by at least 2 C atoms,
or
a group selected from among pyrrolidin-2-yl-C₁₋₄-alkyl-O-, pyrrolidin-3-yl-C₁₋₄-alkyl-O-, piperidin-2-yl-C₁₋₄-alkyl-O-, piperidin-3-yl-C₁₋₄-alkyl-O-, piperidin-4-yl-C₁₋₄-alkyl-O-, azepan-2-yl-C₁₋₄-alkyl-O-, azepan-3-yl-C₁₋₄-alkyl-O-, azepan-4-yl-C₁₋₄-alkyl-O-, morpholin-2-yl-C₁₋₄-alkyl-O-, morpholin-3-yl-C₁₋₄-alkyl-O-, 1-(C₁₋₃-alkyl)-pyrrolidin-2-yl-C₁₋₄-alkyl-O-, 1-(C₁₋₃-alkyl)-pyrrolidin-3-yl-C₁₋₄-alkyl-O-, 1-(C₁₋₃-alkyl)-piperidin-2-yl-C₁₋₄-alkyl-O-, 1-(C₁₋₃-alkyl)-piperidin-3-yl-C₁₋₄-alkyl-O,- 1-(C₁₋₃-alkyl)-piperidin-4-yl-C₁₋₄-alkyl-O-, 1-(C₁₋₃-alkyl)-azepan-2-yl-C₁₋₄-alkyl-O-, 1-(C₁₋₃-alkyl)-azepan-3-yl-C₁₋₄-alkyl-O-, 1-(C₁₋₃-alkyl)-azepan-4-yl-C₁₋₄-alkyl-O-, 4-(C₁₋₃-alkyl)-morpholin-2-yl-C₁₋₄-alkyl-O- and 4-(C₁₋₃-alkyl)-morpholin-3-yl-C₁₋₄-alkyl-O-,
wherein
**R⁴** denotes a group, which may be identical or different, selected from among OH, C₁₋₃-alkyl-O-, C₃₋₆-cycloalkyl-O-, NH₂, C₁₋₃-alkyl-NH-, (C₁₋₃-alkyl)₂N-, (2-methoxyethyl)₂N-, pyrrolidin-1-yl-, piperidin-1-yl-, azepan-1-yl-, morpholin-4-yl-, 1,4-oxazepan-4-yl-, 2-oxa-5-aza-bicyclo[2,2,1]hept-5-yl-, 3-oxa-8-aza-bicyclo[3.2.1]oct-8-yl-, 8-oxa-3-aza-bicyclo[3.2.1]oct-3-yl-, piperazin-1-yl-, 4-(C₁₋₃-alkyl)-piperazin-1-yl-, 1,4-diazepan-1-yl-, 4-(C₁₋₃-alkyl)-1,4-diazepan-1-yl, HCO-NH-, C₁₋₄-alkyl-CO-NH-, C₁₋₃-alkyl-O-C₁₋₃-alkyl-CO-NH-, C₁₋₄-alkyl-O-CO-NH-, H₂NCONH-, C₁₋₃-alkyl-NH-CO-NH-, (C₁₋₃-alkyl)₂N-CONH-, pyrrolidin-1-yl-CO-NH-, piperidin-1-yl-CO-NH-, piperazin-1-yl-CO-NH-, 4-(C₁₋₃-alkyl)-piperazin-1-yl-CO-NH-, morpholin-4-yl-CO-NH-and C₁₋₄-alkyl-SO₂-NH-,
wherein the pyrrolidinyl, piperidinyl, azepan-1-yl, piperazinyl, 1,4-diazepan-1-yl, morpholinyl and 1,4-oxazepan-4-yl groups mentioned above in the definition of the group R^{d} may each additionally be substituted by one or two C₁₋₃-alkyl groups,
and
wherein the phenyl groups mentioned above in the definition of the group R^{d} are mono- or disubstituted by groups R⁵, wherein
**R⁵** denotes hydrogen, or a group, which may be identical or different, selected from among F, Cl, Br, I, OH, CN, C₁₋₃-alkyl-, C₁₋₃-alkyl-O-, CHF₂, CF₃, -O-CHF₂ and -O-CF₃,
And
unless stated otherwise, the above-mentioned alkyl groups may be straight-chain or branched,
**A** denotes -CO- or -C₁-C₃-alkylene, wherein the -C₁-C₃-alkylene group may be 1-, 2-, 3- or 4-substituted by a group **R⁶,**
**and**
**R⁶** which may be identical or different, denotes hydrogen, or a group selected from among OH, C₁-C₄-alkyl and -O-C₁-C₄-alkyl,
optionally in the form of the tautomers, the racemates, the enantiomers, the diastereomers and the mixtures thereof, and optionally the pharmacologically acceptable acid addition salts thereof.

2. Compounds according to claim 1,
**characterised in that**
**R^{a}** denotes a group selected from among a 3-chloro-2-fluoro-phenyl, 3-chloro-4-fluoro-phenyl, 5-chloro-2-fluoro-phenyl, 2-fluoro-3-methyl-phenyl, 2-fluoro-5-methyl-phenyl, 4-fluoro-3-methyl-phenyl and 3-chloro-2-methyl-phenyl group,
**R^{b} and R^{c}** which may be identical or different, denote hydrogen or C₁₋₃-alkyl,
**R^{d}** denotes C₁₋₃-alkyl-O-,
unless stated otherwise, the above-mentioned alkyl groups may be straight-chain or branched,
**A** denotes -CH₂CH₂-, wherein the -CH₂CH₂- group may be substituted by 1 or 2 methyl groups,
optionally in the form of the tautomers, the racemates, the enantiomers, the diastereomers and the mixtures thereof, and optionally the pharmacologically acceptable acid addition salts thereof.

3. Compound 1 according to claim 1 or 2, 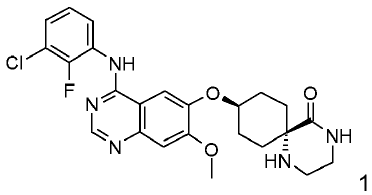 optionally in the form of the tautomers, the racemates, the enantiomers, the diastereomers and the mixtures thereof, and optionally the pharmacologically acceptable acid addition salts thereof.

4. Compound 1 (1) according to claim 1 or 2, 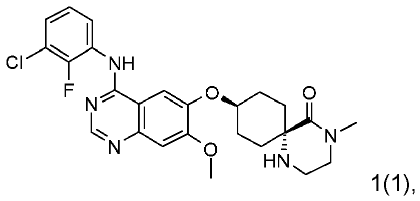 optionally in the form of the tautomers, the racemates, the enantiomers, the diastereomers and the mixtures thereof, and optionally the pharmacologically acceptable acid addition salts thereof.

5. Compound 1 (2) according to claim 1 or 2, 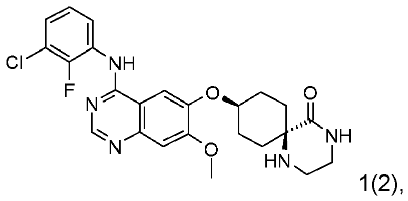 optionally in the form of the tautomers, the racemates, the enantiomers, the diastereomers and the mixtures thereof, and optionally the pharmacologically acceptable acid addition salts thereof.

6. Compound 2 according to claim 1 or 2, 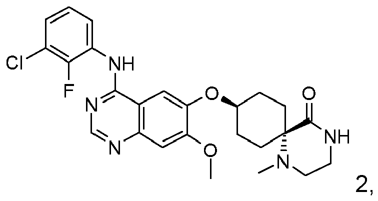 optionally in the form of the tautomers, the racemates, the enantiomers, the diastereomers and the mixtures thereof, and optionally the pharmacologically acceptable acid addition salts thereof.

7. Compound 2(1) according to claim 1 or 2, 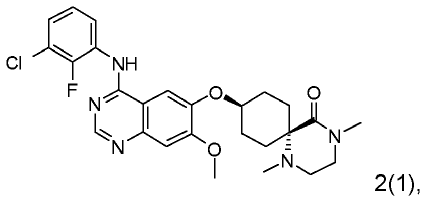 optionally in the form of the tautomers, the racemates, the enantiomers, the diastereomers and the mixtures thereof, and optionally the pharmacologically acceptable acid addition salts thereof.

8. Compound 3 according to claim 1 or 2, 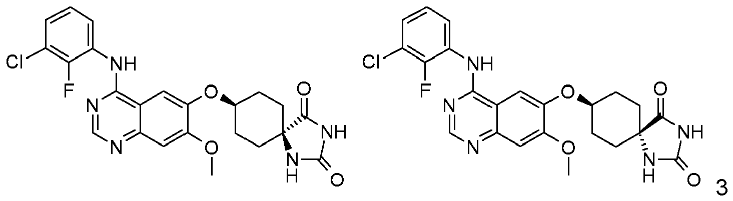 optionally in the form of the tautomers, the racemates, the enantiomers, the diastereomers and the mixtures thereof, and optionally the pharmacologically acceptable acid addition salts thereof.

9. Compounds of formula (I) according to one of claims 1 to 8 for use as medicaments.

10. Compounds of formula (I) according to one of claims 1 to 8 for treating inflammatory or allergic diseases of the airways.

11. Compounds according to claim 10 for the treatment of a disease selected from among chronic bronchitis, acute bronchitis, bronchitis caused by bacterial or viral infection or fungi or helminths, allergic bronchitis, toxic bronchitis, chronic obstructive bronchitis (COPD), asthma (intrinsic or allergic), paediatric asthma, bronchiectasis, allergic alveolitis, allergic or non-allergic rhinitis, chronic sinusitis, cystic fibrosis or mucoviscidosis, alpha-1-antitrypsin deficiency, cough, pulmonary emphysema, interstitial lung diseases, alveolitis, hyperreactive airways, nasal polyps, pulmonary oedema, pneumonitis of different origins, e.g. radiation-induced or caused by aspiration or infectious pneumonitis, collagenoses such as lupus erythematodes, systemic scleroderma, sarcoidosis and Boeck's disease.

12. Compounds of formula (I) according to one of claims 1 to 8, for the treatment of inflammatory or allergic conditions in which autoimmune reactions are involved.

13. Compounds of formula (I) according to one of claims 1 to 8, for the treatment of benign or malignant tumours.

14. Pharmaceutical formulation containing a compound of formula (I) according to one of claims 1 or 8.

15. Orally administered pharmaceutical formulation according to claim 14 containing a compound of formula (I) according to claim 1 to 8.

16. Medicament combinations which contain, besides one or more compounds of formula (I) according to one of claims 1 to 8, as a further active substance, one or more compounds selected from among the categories of betamimetics, anticholinergics, corticosteroids, further PDE4-inhibitors, LTD4-antagonists, EGFR-inhibitors, dopamine agonists, H1-antihistamines, PAF-antagonists and PI3-kinase inhibitors or double or triple combinations thereof.

## Revendications

1. Composés de formule générale (I) 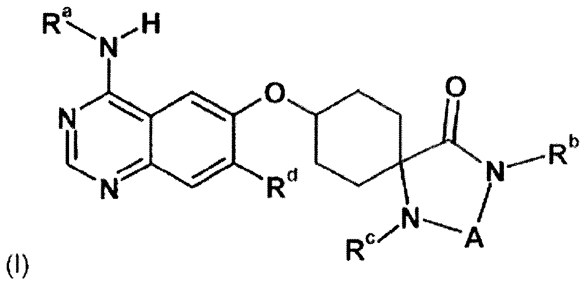 **caractérisés en ce que**
R^{a} est un groupe phényle ou 1-phényléthyle dans lequel le noyau phényle est substitué respectivement par les radicaux R¹ à R³, où
R¹ et R² désignent, identiques ou différents, un atome d'hydrogène ou
un radical choisi dans le groupe comprenant F, Cl, Br, I, OCH₂F, OCHF₂, OCF₃, CH₂F, CHF₂, CF₃, CN, NO₂, NH₂ et OH
ou
un radical choisi dans le groupe comprenant un groupe alkyle en C₁ à C₄, alkyle en C₁ à C₄-O-, alcényle en C₂ à C₃, alcinyle en C₂ à C₃, phényle, phényl-O-, phényl-alkyle en C₁ à C₃, phényl-alkyle en C₁ à C₃-O-, hétéroaryle, hétéroaryl-O-, hétéroaryl-alkyle en C₁ à C₃, hétéroaryl-alkyle en C₁ à C₃-O-, les groupes phényle précédemment cités étant mono- ou disubstitués par les radicaux R⁵,
et
R³ est un atome d'hydrogène
ou
un radical choisi dans le groupe comprenant F, Cl, Br et CH₃,
R^{b} est un atome d'hydrogène ou un radical éventuellement substitué, choisi dans le groupe comprenant les groupes alkyle en C₁ à C₆, cycloalkyle en C₃ à C₆ et cycloalkyle en C₃ à C₆-alkyle en C₁ à C₃,
R^{c} est un atome d'hydrogène ou un radical éventuellement substitué, choisi dans le groupe comprenant les groupes alkyle en C₁ à C₆, cycloalkyle en C₃ à C₆, cycloalkyle en C₃ à C₆-alkyle en C₁ à C₃, alkyle en C₁ à C₆-CO-, cycloalkyle en C₃ à C₆-CO-, cycloalkyle en C₃ à C₆-alkyle en C₁ à C₃-CO, alkyle en C₁ à C₆-SO₂, cycloalkyle en C₃ à C₆-SO₂, cycloalkyle en C₃ à C₆-alkyle en C₁ à C₃-SO₂, phényl-CO- et phényl-SO₂,
R^{d} est un atome d'hydrogène ou
un radical choisi dans le groupe comprenant
F, Cl, Br, I, OH, un groupe alkyle en C₁ à C₄, alkyle en C₁ à C₄-O-, un groupe alkyle en C₁ à C₂-O-substitué par 1 à 3 atomes de fluor, cycloalkyle en C₃ à C₇-O-, cycloalkyle en C₃ à C₇-alkyle en C1 à C₄-O, tétrahydrofuran-3-yl-O-, tétrahydropyran-3-yl-O-, tétrahydro-pyran-4-yl-O-, tétrahydrofuranyl-alkyle en C₁ à C₄-O- et tétrahydropyranyl-alkyle en C₁ à C₄-O-,
ou
R⁴-alkyle en C₁ à C₄, la liaison des radicaux R⁴ pouvant s'effectuer par chaque atome de carbone du radical alkyle,
ou
R⁴-alkyle en C₂ à C₄-alkyl-O-, le radical R⁴ étant séparé de l'atome d'oxygène par au moins 2 atomes de carbone,
ou
un radical choisi dans le groupe comprenant
les groupes pyrrolidin-2-yl-alkyle en C₁ à C₄-O-, pyrrolidin-3-yl-alkyle en C₁ à C₄-O-, pipéridin-2-yl-alkyle en C₁ à C₄-O-, pipéridin-3-yl-alkyle en C₁ à C₄-0-, pipéridin-4-yl-alkyle en C₁ à C₄-O-, azépan-2-yl-alkyle en C₁ à C₄-O-, azépan-3-yl-alkyle en C₁ à C₄-O-, azépan-4-yl-alkyle en C₁ à C₄-O-, morpholin-2-yl-alkyle en C₁ à C₄-O-, morpholin-3-yl-alkyle en C₁ à C₄-O-, 1-(alkyle en C₁ à C₃)-pyrrolidin-2-yl-alkyle en C₁ à C₄-O-, 1- (alkyle en C₁ à C₃) -pyrrolidin-3-yl-alkyle en C₁ à C₄-O-, 1- (alkyle en C₁ à C₃) -pipéridin-2-yl-alkyle en C₁ à C₄-O-, 1- (alkyle en C₁ à C₃) -pipéridin-3-yl-alkyle en C₁ à C₄-O-, 1- (alkyle en C₁ à C₃) -pipéridin-4-yl-alkyle en C₁ à C₄-O-, 1- (alkyle en C₁ à C₃) -azépan-2-yl-alkyle en C₁ à C₄-O-, 1- (alkyle en C₁ à C₃) -azépan-3-yl-alkyle en C₁ à C₄-O-, 1- (alkyle en C₁ à C₃) -azépan-4-yl-alkyle en C₁ à C₄-O, 4-(alkyle en C₁ à C₃) -morpholin-2-yl-alkyle en C₁ à C₄-O- et 4- (alkyle en C₁ à C₃) -morpholin-3-yl-alkyle en C₁ à C₄-O-,
où
les R⁴ désignent un radical, identique ou différent, choisi dans le groupe comprenant OH, les groupes alkyle en C₁ à C₃-O-, cycloalkyle en C₃ à C₆-O-, NH₂, alkyle en C₁ à C₃-NH-, (alkyle en C₁ à C₃)₂N-, (2-méthoxyéthyl)₂N-, pyrrolidin-1-yle, pipéridin-1-yle, azépan-1-yle, morpholin-4-yle, 1,4-oxazépan-4-yle, 2-oxa-5-aza-bicyclo[2.2.1]hept-5-yl, 3-oxa-8-aza-bicyclo[3.2.1]oct-8-yle, 8-oxa-3-aza-bicyclo[3.2.1]oct-3-yle, pipérazin-1-yle, 4-(alkyle en C₁ à C₃)-pipérazin-1-yle, 1,4-diazépan-1-yle, 4-(alkyle en C₁ à C₃)-1,4-diazépan-1-yle, HCO-NH, alkyle en C₁ à C₄-CO-NH, alkyle en C₁ à C₃-O-alkyle en C₁ à C₃-CO-NH, alkyle en C₁ à C₄-O-CO-NH-, H₂NCONH-, alkyle en C₁ à C₃-NH-CO-NH-, (alkyle en C₁ à C₃)₂N-CONH, pyrrolidin-1-yl-CO-NH-, pipéridin-1-yl-CO-NH-, pipérazin-1-yl-CO-NH-, 4-(alkyle en C₁ à C₃)-pipérazin-1-yl-CO-NH-, morpholin-4-yl-CO-NH et alkyle en C₁ à C₄-SO₂-NH-,
les groupes pyrrolidinyle, pipéridinyle, azépan-1-yle, pipérazinyle, 1,4-diazépan-1-yle, morpholinyle et 1,4-oxazépan-4-yle précédemment cités pour la définition du radical R^{d} pouvant en outre être substitués respectivement par un ou deux groupes alkyle en C₁ à C₃,
et
les groupes phényle précédemment cités pour la définition du radical R^{d} sont mono-, ou disubstitués par des radicaux R⁵, où
R⁵ est un atome d'hydrogène, ou
un radical, identique ou différent, choisi dans le groupe comprenant F, Cl, Br, I, OH, CN, alkyle en à C₁ à C₃, alkyle en C₁ à C₃-O-, CHF₂, CF₃, -O-CHF₂ et -O-CF₃,
et
sauf indication contraire, les groupes alkyle mentionnés précédemment peuvent être à chaîne linéaire ou ramifiés,
A est un groupe -CO- ou alkylène en C₁ à C₃,
le radical alkylène en C₁ à C₃ pouvant être substitué 1, 2, 3 ou 4 fois par un radical R⁶,
et
R⁶, identique ou différent, désigne un atome d'hydrogène ou
un radical choisi dans le groupe comprenant OH, un groupe alkyle en C₁ à C₄ et -O-alkyle en C₁ à C₄,
éventuellement sous la forme de leurs tautomères, leurs racémates, leurs énantiomères, leurs diastéréomères et leurs mélanges et éventuellement leurs sels d'addition acide pharmacologiquement acceptables.

2. Composés selon la revendication 1,
**caractérisé en ce que**
R^{a} est un radical choisi dans le groupe comprenant un groupe 3-chloro-2-fluoro-phényle, 3-chloro-4-fluoro-phényle, 5-chloro-2-fluoro-phényle, 2-fluoro-3-méthyl-phényle, 2-fluoro-5-méthyl-phényle, 4-fluoro-3-méthyl-phényle et 3-chloro-2-méthyl-phényle,
R^{b} et R^{c} sont, identiques ou différents
un atome d'hydrogène ou un groupe alkyle en C₁ à C₃,
R^{d} est un groupe alkyle en C₁ à C₃-O-sauf indication contraire, les groupes alkyle mentionnés précédemment pouvant être à chaîne linéaire ou ramifiée,
A est -CH₂CH₂-, le radical CH₂CH₂ pouvant être substitué par 1 ou 2 groupes méthyle,
éventuellement sous la forme de leurs tautomères, leurs racémates, leurs énantiomères, leurs diastéréomères et leurs mélanges et éventuellement leurs sels d'addition acide pharmacologiquement acceptables.

3. Composé 1 selon la revendication 1 ou 2 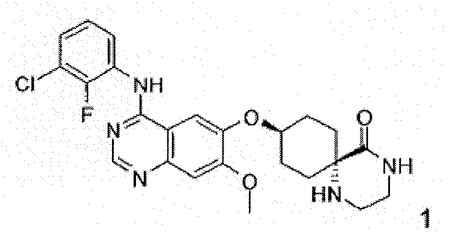 éventuellement sous la forme de leurs tautomères, leurs racémates, leurs énantiomères, leurs diastéréomères et leurs mélanges et éventuellement leurs sels d'addition acide pharmacologiquement acceptables.

4. Composé 1(1) selon la revendication 1 ou 2, 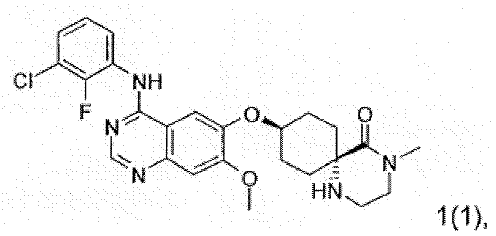 éventuellement sous la forme de leurs tautomères, leurs racémates, leurs énantiomères, leurs diastéréomères et leurs mélanges et éventuellement leurs sels d'addition acide pharmacologiquement acceptables.

5. Composé 1(2) selon la revendication 1 ou 2, 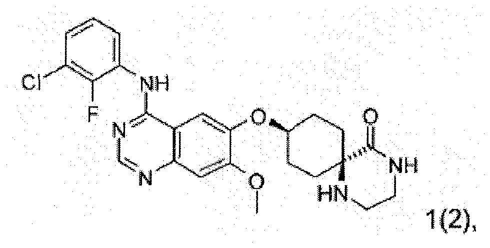 éventuellement sous la forme de leurs tautomères, leurs racémates, leurs énantiomères, leurs diastéréomères et leurs mélanges et éventuellement leurs sels d'addition acide pharmacologiquement acceptables.

6. Composé 2 selon la revendication 1 ou 2 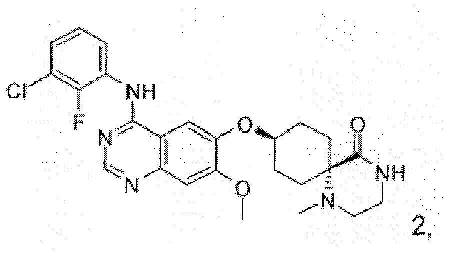 éventuellement sous la forme de leurs tautomères, leurs racémates, leurs énantiomères, leurs diastéréomères et leurs mélanges et éventuellement leurs sels d'addition acide pharmacologiquement acceptables.

7. Composé 2(1) selon la revendication 1 ou 2, 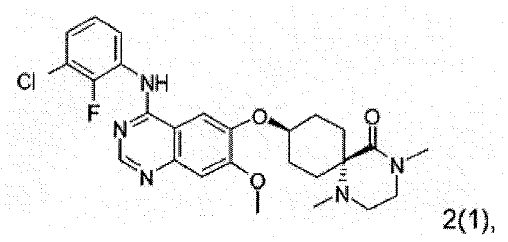 éventuellement sous la forme de leurs tautomères, leurs racémates, leurs énantiomères, leurs diastéréomères et leurs mélanges et éventuellement leurs sels d'addition acide pharmacologiquement acceptables.

8. Composé 3 selon la revendication 1 ou 2, 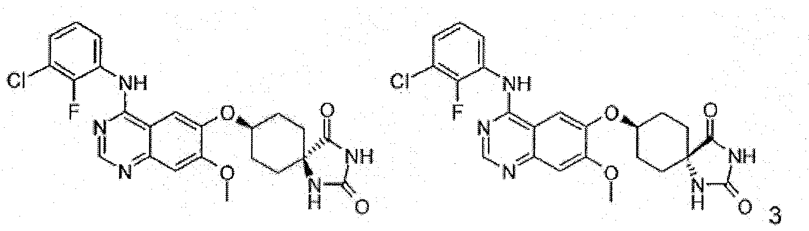 éventuellement sous la forme de leurs tautomères, leurs racémates, leurs énantiomères, leurs diastéréomères et leurs mélanges et éventuellement leurs sels d'addition acide pharmacologiquement acceptables.

9. Composés de formule (I) selon l'une des revendications 1 à 8, pour l'utilisation comme médicament.

10. Composés de formule (I) selon l'une des revendications 1 à 8, pour le traitement des maladies inflammatoires ou allergiques des voies respiratoires.

11. Composés selon la revendication 10, pour le traitement d'une maladie qui est choisie dans le groupe comprenant la bronchite chronique, la bronchite aiguë, la bronchite due à une infection bactérienne ou virale ou à des champignons ou à des vers, une bronchite allergique, une bronchite toxique, une broncho-pneumopathie chronique obstructive (BPCO), un asthme (intrinsèque ou allergique), un asthme pédiatrique, des bronchiectasies, une alvéolite allergique, une rhinite allergique ou non allergique, une sinusite chronique, une fibrose kystique ou une mucoviscidose, un déficit en antitrypsine alpha-1, une toux, un emphysème pulmonaire, des maladies pulmonaires interstitielles, une alvéolite, une hyperactivité des voies respiratoires, des polypes nasaux, les oedèmes pulmonaires, une pneumopathie d'origines diverses telle que induite par des rayons ou par aspiration ou d'origine infectieuse, des collagénoses telles que le lupus érythémateux, la sclérodermie systémique, la sarcoïdose et la maladie de Boeck.

12. Composés de formule (I) selon l'une des revendications 1 à 8, pour le traitement d'états pathologiques inflammatoires ou allergiques dans lesquels des réactions auto-immunes sont impliquées.

13. Composés de formule (I) selon l'une des revendications 1 à 8 pour le traitement des tumeurs bénignes ou malignes.

14. Formulation pharmaceutique contenant un composé de formule (I) selon l'une des revendications 1 ou 8.

15. Formulation pharmaceutique utilisable par voie orale selon la revendication 14, contenant un composé de formule (I) selon les revendications 1 à 8.

16. Combinaisons de médicaments qui contiennent, en plus d'un ou plusieurs composés de formule (I) selon l'une des revendications 1 à 8, comme autre substance active, un ou plusieurs composés qui sont choisis dans les classes des bêtamimétiques, anti-cholinergiques, corticoïdes, d'autres inhibiteurs de PDE-4, des antagonistes de LTD4, des inhibiteurs de l'EGFR, des agonistes de la dopamine, des antihistaminiques H1, des antagonistes de PAF et des inhibiteurs de la P13 kinase ou des combinaisons doubles ou triples de ceux-ci.
